(19) Europäisches Patentamt
European Patent Office
Office européen des brevets

(11) EP 3 493 815 B1

(12) **EUROPEAN PATENT SPECIFICATION**

(45) Date of publication and mention of the grant of the patent:
**16.02.2022 Bulletin 2022/07**

(21) Application number: **17837680.2**

(22) Date of filing: **03.08.2017**

(51) International Patent Classification (IPC):
***A61K 31/675*** (2006.01) ***A61K 31/5377*** (2006.01)
***A61K 38/38*** (2006.01) ***A61K 47/42*** (2017.01)
*A61K 9/08* (2006.01) *A61K 9/19* (2006.01)

(52) Cooperative Patent Classification (CPC):
(C-Sets available)
**A61K 9/0019; A61K 9/08; A61K 9/19; A61K 31/5377; A61K 31/675; A61K 38/385; A61K 47/42** (Cont.)

(86) International application number:
**PCT/US2017/045290**

(87) International publication number:
**WO 2018/027029 (08.02.2018 Gazette 2018/06)**

(54) **FORMULATIONS OF FOSAPREPITANT AND APREPITANT**

FORMULIERUNGEN VON FOSAPREPITANT UND APREPITANT

FORMULE DE FOSAPRÉPITANT ET D'APRÉPITANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2016 US 201662370453 P**
**24.10.2016 US 201662412085 P**

(43) Date of publication of application:
**12.06.2019 Bulletin 2019/24**

(73) Proprietor: **Zhuhai Beihai Biotech Co., Ltd.**
**Zhuhai (CN)**

(72) Inventor: **SUN, Qun**
**Princeton, New Jersey 08540 (US)**

(74) Representative: **Fish & Richardson P.C.**
**Highlight Business Towers**
**Mies-van-der-Rohe-Straße 8**
**80807 München (DE)**

(56) References cited:
**WO-A1-2011/019911** **WO-A1-2014/093907**
**WO-A1-2016/059587** **US-A1- 2004 014 655**
**US-A1- 2013 317 016** **US-A1- 2015 165 045**
**US-A1- 2015 366 984**

- **PATRICK LANGFORD ET AL: "Fosaprepitant and aprepitant: an update of the evidence for their place in the prevention of chemotherapy-induced nausea and vomiting", CORE EVIDENCE, vol. 5, 24 September 2009 (2009-09-24), pages 77-90, XP055570918, DOI: 10.2147/CE.S6012**
- **SUSANA GARCIA-RECIO ET AL: "Biological and Pharmacological Aspects of the NK1-Receptor", BIOMED RESEARCH INTERNATIONAL, vol. 2015, 1 January 2015 (2015-01-01), pages 1-14, XP055601325, ISSN: 2314-6133, DOI: 10.1155/2015/495704**
- **T. L. NG ET AL: "Chemotherapy-Induced Nausea and Vomiting: Time for More Emphasis on Nausea?", THE ONCOLOGIST, vol. 20, no. 6, 6 May 2015 (2015-05-06), pages 576-583, XP055601329, US ISSN: 1083-7159, DOI: 10.1634/theoncologist.2014-0438**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/5377, A61K 2300/00;**
**A61K 31/675, A61K 2300/00;**
**A61K 38/385, A61K 2300/00**

## Description

### CLAIM OF PRIORITY

This application claims the benefit of U.S. provisional application No. 62/370,453 filed August 3, 2016 and U.S. provisional application No. 62/412,085 filed October 24, 2016.

### TECHNICAL FIELD

This document relates to compositions and formulations for the treatment and prevention of chemotherapy-induced nausea and vomiting, and more particularly to compositions comprising fosaprepitant, or a pharmaceutically acceptable salt thereof, and aprepitant.

### BACKGROUND

Nausea and vomiting are common complications of cancer chemotherapy. Patients consistently report that chemotherapy-induced nausea and vomiting (CINV) is an aspect of treatment they find most unpleasant and distressing. This syndrome has a significant impact on patients' functional status and quality of life and patients may delay scheduled chemotherapy or even on occasion refuse potentially curative therapy because of CINV.

Aprepitant is a selective high-affinity antagonist at human substance P neurokinin 1 (NK1) receptors used for the prevention of CINV. Despite the demonstrated benefits of oral aprepitant, there is still a medical need for treatment options (such as intravenous administration) to prevent CINV in patients who cannot easily tolerate orally administered medication prior to initiating chemotherapy. Parenteral administration is also an important treatment option for oncologists for whom it is frequently more convenient and easier to administer compounds intravenously prior to the administration of chemotherapy (which is also commonly given intravenously).

Fosaprepitant is a water soluble phosphoryl prodrug to aprepitant. Following intravenous administration, fosaprepitant is rapidly converted to aprepitant *in vivo* via ubiquitous phosphatases. The antiemetic effects of fosaprepitant are attributed to aprepitant.

Aprepitant is insoluble in water whereas fosaprepitant is soluble in water. Fosaprepitant easily degrades to aprepitant unless stored at low temperature. Degradation is enhanced by the presence of water. To solubilise possibly present aprepitant, current fosaprepitant formulation contains polysorbate 80. Polysorbate 80 may cause hypersensitivity reactions, including flushing, itching or shortness of breath, and has the potential to cause severe anaphylaxis reactions, and cause infusion site reactions (Leal AD et al., Support Care Cancer 2014; 22:1313-1317).

There is a need for the fosaprepitant or aprepitant formulations containing no polysorbate 80 that are suitable for parenteral administration.

Langford P. et al. describe in Core Evidence, vol. 5, 24 September 2009, on pages 77-90 the evidence regarding the use of fosaprepitant and aprepitant in the prevention of chemotherapy-induced nausea and vomiting.

WO 2014/093907 A1 describes sustained release pharmaceutical formulations which can deliver a 5-hydroxytryptamine 3 receptor antagonist and a neurokinin-1 receptor antagonist, the latter of which can be aprepitant or fosaprepitant.

WO 2016/059587 A1 describes stable, non-aqueous and ready-to-use injectable compositions of a pharmaceutically active agents including fosaprepitant.

### SUMMARY

The present invention is defined by the independent claims. The dependent claims depict additional embodiments of the invention.

Also, provided herein is a composition comprising fosaprepitant, or a pharmaceutically acceptable salt thereof, aprepitant and human serum albumin. In some embodiments, the fosaprepitant, or a pharmaceutically acceptable salt thereof, and the human serum albumin in the composition have a ratio by weight from about 1:0.5 to about 1:300.

In some embodiments, the aprepitant and the fosaprepitant, or a pharmaceutically acceptable salt thereof, in the composition have a ratio by weight no more than 1:100 or no more than 1:200.

In some embodiments, the fosaprepitant, or the pharmaceutically acceptable salt thereof, and the human serum albumin in the composition have a ratio by weight from about 1:1 to about 1:150. In some embodiments, the composition is an aqueous formulation. In some embodiments, the aqueous formulation is substantially free of solvent other than water. In some embodiments, the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

Also, provided herein is a liquid pharmaceutical composition comprising the composition comprising the fosaprepitant, or the pharmaceutically acceptable salt thereof, the aprepitant and the human serum albumin as described herein, and a pharmaceutically acceptable carrier. The liquid pharmaceutical composition is an aqueous solution substantially free of solvent other than water.

In some embodiments, the liquid pharmaceutical composition is a reconstituted solution, reconstituted from the solid composition comprising the fosaprepitant, or the pharmaceutically acceptable salt thereof, the aprepitant and the human serum albumin as described herein.

In some embodiments, the liquid pharmaceutical composition is an aqueous reconstituted solution, prepared by reconstituting a solid composition comprising the fosaprepitant, or the pharmaceutically acceptable salt thereof, and the aprepitant in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or the pharmaceutically acceptable salt thereof, and the aprepitant in an aqueous solution of human serum albumin, in which the concentration of human serum albumin in the solution is in the range from 0.1% to 25% (w/v).

In some embodiments, the liquid pharmaceutical composition is a clear aqueous solution.

Also, provided herein is a kit comprising a solid composition comprising the fosaprepitant, or the pharmaceutically acceptable salt thereof, and the aprepitant, or the pharmaceutically acceptable salt thereof, and an aqueous solution of human serum albumin. In some embodiments, the solid composition in the kit comprises about 245.3 mg of fosaprepitant dimeglumine. In some embodiments, the aqueous solution of human serum albumin in the kit has concentration of human serum albumin in the range from 0.1% to 25% (w/v). Also provided herein is a kit comprising a solid composition comprising fosaprepitant, or a pharmaceutically acceptable salt thereof, and an aqueous solution of human serum albumin. In some embodiments, the solid composition in the kit comprises 245.3 mg of fosaprepitant dimeglumine equivalent to 150 mg of fosaprepitant free acid. In some embodiments, the aqueous solution of human serum albumin in the kit has concentration of human serum albumin in the range from 0.1% to 25% (w/v). In some embodiments, the solid composition comprises 245.3 mg of fosaprepitant dimeglumine equivalent to 150 mg of fosaprepitant free acid and an aqueous solution of human serum albumin with the human serum albumin concentration in the range from 0.1% to 25% (w/v).

DETAILED DESCRIPTION

As used herein the term "aprepitant" is a compound that has the CAS No. 170729-80-3 and the following chemical structure:

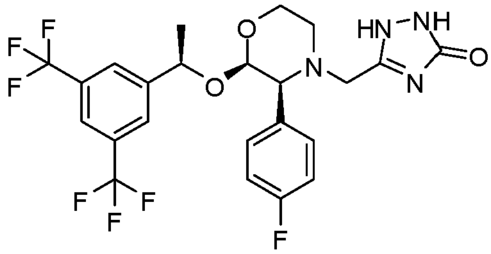

Aprepitant is a white to off-white crystalline solid, with a molecular weight of 534.43. It is practicallyinsoluble in water. Aprepitant is sparingly soluble in ethanol and isopropyl acetate and slightly soluble inacetonitrile.

Aprepitant (EMEND) is a substance P/neurokinin 1 (NK1) receptor antagonist, indicated in combination with other antiemetic for prevention of acute and delayed nausea and vomiting associated with initial and repeat courses of highly emetogenic cancer chemotherapy (HEC) including high-dose cisplatin, and nausea and vomiting associated with initial and repeat courses of moderately emetogenic cancer chemotherapy (MEC). Aprepitant is also indicated for prevention of postoperative nausea and vomiting (PONV) in adults.

In some embodiments, the aprepitant can be a pharmaceutically acceptable salt of fosaprepitant. In some embodiments, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally

capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; aminosugars such as meglumine, and amino acids such as arginine, lysine, and the like.

**Composition comprising fosaprepitant, or a pharmaceutically acceptable salt thereof, aprepitant and human serum albumin.**

Also, provided herein is a composition comprising fosaprepitant, or a pharmaceutically acceptable salt thereof, aprepitant and human serum albumin. In some embodiments, the fosaprepitant, or a pharmaceutically acceptable salt thereof, and the human serum albumin in the composition have a ratio by weight from about 1:0.5 to about 1:300.

In some embodiments, the aprepitant and the fosaprepitant, or a pharmaceutically acceptable salt thereof, in the composition have a ratio by weight no more than 1:100, no more than 1:200, no more than 1:500, or no more than 1:1000. In some embodiments, the weight ratio of fosaprepitant, or a pharmaceutically acceptable salt thereof, to aprepitant in the composition is from about 1000:1 to about 20:1, from about 1000:1 to about 50:1, from about 1000:1 to about 70:1, or from about 1000:1 to about 100:1.

Fosaprepitant easily degrades to aprepitant unless stored at low temperature. Degradation is enhanced by the presence of water. In some embodiments, all or part of aprepitant in the composition is degraded from fosaprepitant, or a pharmaceutically acceptable salt thereof. In some embodiments, all aprepitant in the composition is degraded from fosaprepitant, or a pharmaceutically acceptable salt thereof. In some embodiments, part of aprepitant in the composition is degraded from fosaprepitant, or a pharmaceutically acceptable salt thereof.

In some embodiments, the fosaprepitant, or the pharmaceutically acceptable salt thereof, and the human serum albumin in the composition have a ratio by weight from about 1:1 to about 1:150, from about 1:2 to about 1:100, from about 1:2 to about 1:80, from about 1:2 to about 1:50, from about 1:2.5 to about 1:30, from about 1:3 to about 1:20, or from about 2:1 to about 1:20. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, human serum albumin refers to native and recombinant human serum albumin. Native human serum albumin and other plasma proteins can be precipitated from human plasma by varying the pH and adding ethanol, in what is known as the Cohn fractionation process (Cohn EJ et al., J. Am. Chem. Soc. 1946; 68:459-475). By controlling the pH and ethanol content, semi-purified fractions of plasma proteins can be produced. One of the last proteins to precipitate in the Cohn process is native human serum albumin. After precipitation, a wet paste of crude native human serum albumin is obtained. Subsequent bioprocessing steps (purification, filtration, pasteurization, etc.) can be used to produce a purified, stabilized form of native human serum albumin for commercial use (Lin JJ et al., Pharmaceutical Research 2000; 17:391-6). Recombinant human serum albumin is a highly purified animal-, virus-, and prion-free product as alternative to native human serum albumin, to which it is structurally equivalent (Bosse D et al., J. Clin. Pharmacol. 2005; 45:57-67). Recombinant human serum albumin has been produced by various hosts, both prokaryotic and eukaryotic (Chen Z et al., Biochimica et Biophysica Acta 2013; 1830:5515-5525). A fatty acid free human

serum albumin can be prepared by treatment of human serum albumin with charcoal at low pH. Likewise, treatment of human serum albumin with charcoal at low pH can be used to remove fatty acids from human serum albumin (Chen RF, J. Biol. Chem. 1967; 242:173-181). Human serum albumin (HSA) is a highly soluble globular protein of Mr 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (1981), Vorum, Dan. Med. Bull., 46, 379-99 (1999), Kragh-Hansen, Dan. Med Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (1992), and Carter et al., Adv. Protein. Chem., 45, 153-203 (1994)).

In some embodiments, the human serum albumin is a native human serum albumin. In some embodiments, the human serum albumin is a recombinant human serum albumin. In some embodiments, the human serum albumin is a fatty acid free human serum albumin. In some embodiments, the human serum albumin is essentially fatty acid free.

As used herein, the term "essentially fatty acid free" refers to proteins (e.g. serum albumin) that contain less than about 0.02% fatty acid by weight. For example, human serum albumin that is essentially fatty acid free can contain less than 0.02% fatty acid by weight. In some embodiments, the human serum albumin contains no more than 2, 1, 0.5, 0.1, 0.05, 0.01, 0.001, 0.0005, or 0.0001 moles of fatty acids bound to one mole of human serum albumin.

As used herein, the term "fatty acids" refers to non-esterified fatty acids (e.g. linoleic acid, $\alpha$-linoleic acid, $\gamma$-linoleic acid).

Solutions of human serum albumin for infusion are commercially available. Those solutions must be supplemented with stabilizers to allow pasteurization and storage, to avoid the spontaneous polymerization of the albumin. Usually, N-acetyltryptophan and caprylic acid or their sodium salts are used in alone or in combination.

In some embodiments, the human serum albumin is a commercially available solution of human serum albumin USP for infusion. In some embodiments, the solution of human serum albumin USP for infusion is 5% solution of human serum albumin USP (w/v). In some embodiments, the solution of human serum albumin USP for infusion is 20% solution of human serum albumin USP (w/v). In some embodiments, the solution of human serum albumin USP for infusion is 25% solution of human serum albumin USP (w/v). In some embodiments, the human serum albumin is an aqueous solution prepared by diluting a commercially available solution of human serum albumin USP for infusion with saline. In some embodiments, the human serum albumin is an aqueous solution prepared by diluting a commercially available solution of human serum albumin USP for infusion with water.

In some embodiments, the composition comprises at least one stabilizer for the human serum albumin. In some embodiments, the composition comprises two stabilizers for the human serum albumin. In some embodiments, the stabilizers are N-acetyltryptophan and caprylic acid or sodium salt thereof. In some embodiments, the stabilizer is N-acetyltryptophan or sodium salt thereof. In some embodiments, the stabilizer is caprylic acid or sodium salt thereof.

As used herein the term "fosaprepitant" is a compound that has the CAS No. 172673-20-0 and the following chemical structure:

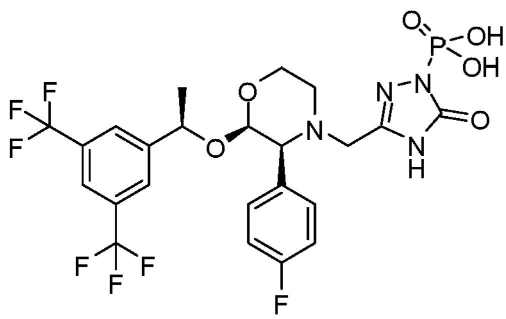

Fosaprepitant (Emend for Injection (US), Ivemend (EU)) is an antiemetic drug, administered intravenously. It is a prodrug of aprepitant.

In some embodiments, the fosaprepitant can be a pharmaceutically acceptable salt of fosaprepitant.

As used herein, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; aminosugars such as meglumine; and amino acids such as arginine, lysine, and the like.

In some embodiments, the fosaprepitant, or a pharmaceutically acceptable salt thereof, can be fosaprepitant dimeglumine, which is a dimeglumine salt of fosaprepitant.

As used herein the term "fosaprepitant dimeglumine" is a compound that has the CAS No. 265121-04-8 and the following chemical structure:

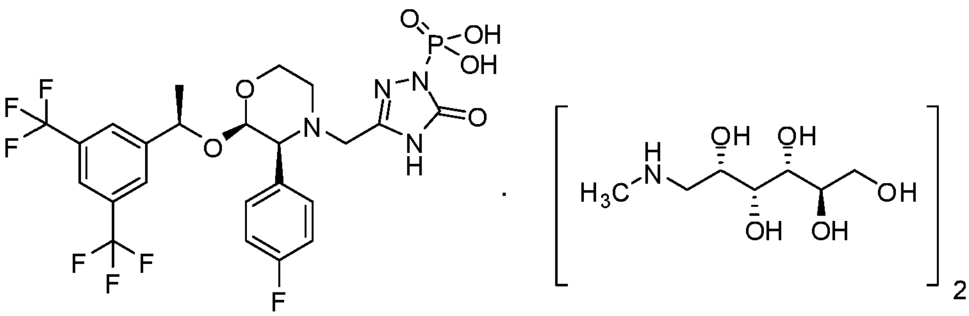

As used herein the term "aprepitant" is a compound that has the CAS No. 170729-80-3 and the following chemical structure:

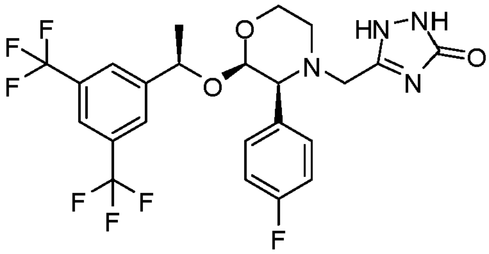

Aprepitant is a white to off-white crystalline solid, with a molecular weight of 534.43. It is practicallyinsoluble in water. Aprepitant is sparingly soluble in ethanol and isopropyl acetate and slightly soluble inacetonitrile.

Aprepitant (EMEND) is a substance P/neurokinin 1 (NK1) receptor antagonist, indicated in combination with other antiemetic for prevention of acute and delayed nausea and vomiting associated with initial and repeat courses of highly emetogenic cancer chemotherapy (HEC) including high-dose cisplatin, and nausea and vomiting associated with initial and repeat courses
of moderately emetogenic cancer chemotherapy (MEC). Aprepitant is also indicated for prevention of postoperative nausea and vomiting (PONV) in adults.

In some embodiments, the aprepitant can be a pharmaceutically acceptable salt of fosaprepitant. In some embodiments, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; aminosugars such as meglumine, and amino acids such as arginine, lysine, and the like.

In some embodiments, the fosaprepitant and the human serum albumin in the composition are bound non-covalently. In some embodiments, the composition comprises a non-covalently bond complex comprising fosaprepitant and the human serum albumin.

In some embodiments, the aprepitant and the human serum albumin in the composition are bound non-covalently. In some embodiments, the composition comprises a non-covalently bond complex comprising aprepitant and the human serum albumin.

In some embodiments, the fosaprepitant is bound non-covalently to the human serum albumin in the composition. In some embodiments, the aprepitant is bound non-covalently to the human serum albumin in the composition.

As used herein, the term "non-covalent" refers to an interaction between two or more components, wherein the bonds between the components are non-covalent bonds (i.e., no atom of one component shares a pair of electrons with an atom of another component; e.g., weak bonds such as hydrogen bonds, electrostatic effects, π-effects, hydrophobic effects and Van der Waals forces). Further, human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Additionally, after the drug molecule binds to HSA, the drug molecule and HSA form a non-covalently bound drug and protein complex through the binding sites of HSA. This concept is commonly understood by one of ordinary skill in the art to which this disclosure belongs. One example of a non-covalently bound complex is a non-covalently bound complex of HSA and fatty acids, in which the fatty acids bind to HSA through HSA's multiple binding sites.

In some embodiments, the non-covalent interaction between fosaprepitant and/or aprepitant, and human serum albumin comprises hydrogen bonding. In some embodiments, the non-covalent interaction between fosaprepitant and/or

aprepitant, and human serum albumin comprises electrostatic interaction. In some embodiments, the non-covalent interaction between fosaprepitant and/or aprepitant, and human serum albumin comprises hydrophobic interaction. In some embodiments, the non-covalent interaction between fosaprepitant and/or aprepitant, and human serum albumin comprises Van der Waals forces. In some embodiments, the non-covalent interaction between fosaprepitant and/or aprepitant, and human serum albumin comprises hydrogen bonding, electrostatic interaction, hydrophobic interaction, and Van der Waals forces.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the composition is an aqueous formulation. In some embodiments, the aqueous formulation is substantially free of solvent other than water. In some embodiments, the aqueous formulation is free of solvent other than water.

As used herein, "substantially free of solvent," in reference to an aqueous solution, refers to an aqueous solution that contains less than 0.5 %, by weight, of any non-water solvent. In some embodiments, the aqueous solution contains less than 0.1%, by weight, of any non-water solvent.

In some embodiments, the aqueous formulation is a clear aqueous solution reconstituted from the solid formulation (e.g. the sterile lyophilized powder) in water. In some embodiments, the aqueous formulation is a clear aqueous solution reconstituted from the solid formulation (e.g. the sterile lyophilized powder) in 0.9% saline. In some embodiments, the aqueous formulation is a clear aqueous solution reconstituted from the solid formulation (e.g. the sterile lyophilized powder) in 5% Dextrose solution.

In some embodiments, the aqueous formulation is a clear aqueous solution reconstituted from the solid formulation (e.g. the sterile lyophilized powder) in water, wherein the aqueous formulation has pH value from about 5 to about 8. In some embodiments, the aqueous formulation is a clear aqueous solution reconstituted from the solid formulation (e.g. the sterile lyophilized powder) in 0.9% saline, wherein the aqueous formulation has pH value from about 5 to about 8. In some embodiments, the aqueous formulation is a clear aqueous solution reconstituted from the solid formulation (e.g. the sterile lyophilized powder) in 5% Dextrose solution, wherein the aqueous formulation has pH value from about 5 to about 8.

In some embodiments, the aqueous formulation is a clear aqueous solution reconstituted from the solid formulation (e.g. the sterile lyophilized powder) in water, wherein the aqueous formulation has pH value from about 6 to about 7.5. In some embodiments, the aqueous formulation is a clear aqueous solution reconstituted from the solid formulation (e.g. the sterile lyophilized powder) in 0.9% saline, wherein the aqueous formulation has pH value from about 6 to about 7.5. In some embodiments, the aqueous formulation is a clear aqueous solution reconstituted from the solid formulation (e.g. the sterile lyophilized powder) in 5% Dextrose solution, wherein the aqueous formulation has pH value from about 6 to about 7.5.

In some embodiments, the aqueous formulation has pH value from about 5 to about 8. In some embodiments, the aqueous formulation has pH value from about 5.5 to about 7.8. In some embodiments, the aqueous formulation has pH value from about 6 to about 7.5. In some embodiments, the aqueous formulation has pH value from about 6.5 to about 7.5. In some embodiments, the aqueous formulation has pH value from about 6 to about 6.5. In some embodiments, the aqueous formulation has pH value from about 6.5 to about 7. In some embodiments, the aqueous formulation has pH value from about 7 to about 7.5. In some embodiments, the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the aqueous formulation is substantially free of solvent other than water. In some embodiments, the aqueous formulation is free of solvent other than water.

In some embodiments, the aqueous formulation is a clear aqueous solution, wherein the aqueous formulation has pH value from about 5 to about 8, and wherein the aqueous formulation is substantially free of solvent other than water. In some embodiments, the aqueous formulation is a clear aqueous solution, wherein the aqueous formulation has pH value from about 5 to about 8, and wherein the aqueous formulation is free of solvent other than water. In some embodiments, the aqueous formulation is a clear aqueous solution, wherein the aqueous formulation has pH value from about 5.5 to about 7.8, and wherein the aqueous formulation is substantially free of solvent other than water. In some embodiments, the aqueous formulation is a clear aqueous solution, wherein the aqueous formulation has pH value from about 5.5 to about 7.8, and wherein the aqueous formulation is free of solvent other than water. In some embodiments, the aqueous formulation is a clear aqueous solution, wherein the aqueous formulation has pH value from about 6 to about 7.5, and wherein the aqueous formulation is substantially free of solvent other than water. In some embodiments, the aqueous formulation is a clear aqueous solution, wherein the aqueous formulation has pH value from about 6 to about 7.5, and wherein the aqueous formulation is free of solvent other than water. In some embodiments, the aqueous formulation is a clear aqueous solution, wherein the aqueous formulation has pH value from about 6.5 to about 7.5, and wherein the aqueous formulation is substantially free of solvent other than water. In some embodiments, the aqueous formulation is a clear aqueous solution, wherein the aqueous formulation has pH value from about 6.5 to about 7.5, and

wherein the aqueous formulation is free of solvent other than water. In some embodiments, the aqueous formulation is a clear aqueous solution, wherein the aqueous formulation has pH value from about 6 to about 6.5, and wherein the aqueous formulation is substantially free of solvent other than water. In some embodiments, the aqueous formulation is a clear aqueous solution, wherein the aqueous formulation has pH value from about 6 to about 6.5, and wherein the aqueous formulation is free of solvent other than water. In some embodiments, the aqueous formulation is a clear aqueous solution, wherein the aqueous formulation has pH value from about 6.5 to about 7, and wherein the aqueous formulation is substantially free of solvent other than water. In some embodiments, the aqueous formulation is a clear aqueous solution, wherein the aqueous formulation has pH value from about 6.5 to about 7, and wherein the aqueous formulation is free of solvent other than water. In some embodiments, the aqueous formulation is a clear aqueous solution, wherein the aqueous formulation has pH value from about 7 to about 7.5, and wherein the aqueous formulation is substantially free of solvent other than water. In some embodiments, the aqueous formulation is a clear aqueous solution, wherein the aqueous formulation has pH value from about 7 to about 7.5, and wherein the aqueous formulation is free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is substantially free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

As used herein, the term "clear aqueous solution" refers to an aqueous solution that is transparent and free of visible particles, precipitation upon visual observation.

When visually observed, for example, the term "clear aqueous solution" excludes a milky aqueous solution. Further, the term "clear aqueous solution" excludes a cloudy or hazy aqueous solution.

In some embodiments, the aqueous solution is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for a period of time selected from 1 hour. 2 hours, 3 hours, 4 hours, 5 hours, 6 hours, 7 hours, 8 hours, 12 hours, and 24 hours. In some embodiments, the aqueous formulation is a clear aqueous solution for at least 24 hours.

Also, provided herein is a pharmaceutical composition comprising the composition comprising the fosaprepitant, or a pharmaceutically acceptable salt thereof, the aprepitant and the human serum albumin as described herein, and a pharmaceutically acceptable carrier. In some embodiments, the pharmaceutical composition comprising the composition comprising the fosaprepitant, or a pharmaceutically acceptable salt thereof, and the human serum albumin as described herein, and a pharmaceutically acceptable carrier, is a liquid pharmaceutical composition. In some embodiments, the liquid pharmaceutical composition is an aqueous solution. In some embodiments, the liquid pharmaceutical composition is free of solvent other than water. In some embodiments, the liquid pharmaceutical composition is substantially free of solvent other than water. In some embodiments, the liquid pharmaceutical composition is an injectable pharmaceutical formulation. In some embodiments, the liquid pharmaceutical composition is a formulation for infusion (e.g., intravenous infusion).

As used herein, the term "aqueous solution" refers to a solution, wherein at least one solvent is water and the weight % of water in the mixture of solvents is at least 50%, at least 60%, at least 70%, at least 90%, at least 95%, or at least 99%. In some embodiments, aqueous solution is a solution in which water is the only solvent. As used herein, the term "aqueous solvent" refer to a liquid comprising at least 50%, at least 60%, at least 70%, at least 90% or at least 95% water. In some embodiments, aqueous solvent is water. In some embodiments, aqueous solvent is saline.

In some embodiments, pharmaceutically acceptable carrier refers to any carrier useful to solubilize and deliver an agent to a subject. A desirable pharmaceutically acceptable carrier is saline. Other pharmaceutically acceptable carrier and their formulation are known to one skilled in the art and described, for example, in Remington's Pharmaceutical Sciences. (20th edition), ed. A. Gennaro, 2003, Lippincon Williams & Wilkins. In some embodiments, the carrier may contain components such as, for example, dextrose, glucose, serum proteins (other than HSA), buffer substances such as phosphates, glycine, sorbic acid, potassium sorbate, salts or electrolytes, such as protamine sulfate, disodium hydrogen phosphate, potassium hydrogen phosphate, sodium chloride, zinc salts, and cellulose-based substances. In some embodiments, the carrier may contain components such as contain anti-oxidants, buffers, bacteriostats and solutes which render the formulation isotonic with the blood of the intended recipient.

In some embodiments, the pharmaceutically acceptable excipient is selected from lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The pharmaceutical compositions may additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The pharmaceutical composition may be formulated so as to provide quick, sustained or delayed release of the active ingredient after administration to the patient by employing procedures known in the art.

In some embodiments, the pharmaceutical composition of the present disclosure may be administered by a syringe or a catheter, or any other means generally known in the art for the delivery of a pharmaceutical agent by injection to the subject in need thereof. The delivery means will vary, as recognized by those skilled in the art, depending on the diseases and conditions treated, the severity of the disease, the sex, age and general health condition of the subject, excipient usage, the possibility of co-usage with other therapeutic treatments such as use of other agents as described herein and the judgment of the treating physician.

The pharmaceutical composition comprising the composition comprising the fosaprepitant, or a pharmaceutically acceptable salt thereof, and the human serum albumin as described herein can be administered to a subject via various routes, such as parenterally, including intravenous, intra-arterial, intraperitoneal, intrapulmonary, oral, inhalation, intravascular, intramuscular, intra-tracheal, subcutaneous, intraocular, intrathecal, or transdermal. For example, the composition can be administered by inhalation to treat conditions of the respiratory tract. In some embodiments, the pharmaceutical composition is administrated intravenously (e.g., as an infusion). In some embodiments, the pharmaceutical composition is administrated orally as a capsule or an oral suspension.

Also, provided herein is a pharmaceutical composition comprising the composition comprising the fosaprepitant dimeglumine, the aprepitant and the human serum albumin as described herein, and a pharmaceutically acceptable carrier.

In some embodiments, the pharmaceutical composition is free of a surfactant, such as CREMOPHOR® surfactants and Polysorbate 80. In some embodiments, the pharmaceutical composition is substantially free of a surfactant, such as CREMOPHOR® surfactants and Polysorbate 80. As used herein, the term "substantially free of surfactant" refers to a formulation containing less than 0.0005%, less than 0.0003%, or less than 0.0001% of surfactants and/or less than 0.0005%, less than 0.0003%, or less than 0.0001% of surfactant.

Also, provided herein is a method for the prevention of chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, the aprepitant and the human serum albumin as described herein, and a pharmaceutically acceptable carrier. In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting is nausea and vomiting associated with initial and repeat courses of moderately emetogenic cancer chemotherapy (MEC). In some embodiments, the nausea and vomiting is postoperative nausea and vomiting (PONV) in adults.

Also, provided herein is a composition for use in a method of treating chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, aprepitant and the human serum albumin as described herein, and a pharmaceutically acceptable carrier.

In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting is nausea and vomiting associated with initial and repeat courses of moderately emetogenic cancer chemotherapy (MEC). In some embodiments, the nausea and vomiting is postoperative nausea and vomiting (PONV) in adults.

As used herein, an "effective amount," "therapeutically effective amount," or a "pharmaceutically-effective amount" in reference to the compounds or compositions of the instant invention refers to the amount sufficient to induce a desired biological, pharmacological, or therapeutic outcome in a subject. That result can be reduction, prevention, mitigation, delay, shortening the time to resolution of, alleviation of the signs or symptoms of, or exert a medically-beneficial effect upon the underlying pathophysiology or pathogenesis of an expected or observed side-effect, toxicity, disorder or condition, or any other desired alteration of a biological system.

In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of delayed nausea and vomiting associated with initial and repeat courses of moderately emetogenic cancer chemotherapy (MEC). In some embodiments, the methods described herein are for the prevention of postoperative nausea and vomiting (PONV) in adults.

As used herein, the terms "individual", "patient", or "subject" are used interchangeably and refer to any animal,

including mammals, preferably mice, rats, other rodents, rabbits, dogs, cats, swine, cattle, sheep, horses, or primates, and most preferably humans.

As used herein the term "treating" or "treatment" refers to 1) inhibiting the disease; for example, inhibiting a disease, condition or disorder in an individual who is experiencing or displaying the pathology or symptomatology of the disease, condition or disorder (i.e., arresting further development of the pathology and/or symptomatology), or 2) ameliorating the disease; for example, ameliorating a disease, condition or disorder in an individual who is experiencing or displaying the pathology or symptomatology of the disease, condition or disorder (i.e., reversing the pathology and/or symptomatology).

As used herein, an "effective amount," "therapeutically effective amount," or a "pharmaceutically-effective amount" in reference to the compounds or compositions of the instant invention refers to the amount sufficient to induce a desired biological, pharmacological, or therapeutic outcome in a subject. That result can be reduction, prevention, mitigation, delay, shortening the time to resolution of, alleviation of the signs or symptoms of, or exert a medically-beneficial effect upon the underlying pathophysiology or pathogenesis of an expected or observed side-effect, toxicity, disorder or condition, or any other desired alteration of a biological system.

As used herein, the term "preventing" (or "prevention") means to completely or almost completely stop an disease or condition (e.g., cancer, metastatic cancer) from occurring, for example when the patient or subject is predisposed to an condition or is at risk of a disease or condition. Preventing can also include inhibiting, i.e., arresting the development, of a condition.

In some embodiments, the methods described herein are performed in combination with at least one other antiemetic agents. In some embodiments, the methods described herein are performed in combination with dexamethasone and a 5-$HT_3$ antagonist (e.g., tropisetron, palonosetron, ramosetron, granisetron, ondansetron, dolasetron, or metoclopramide). In some embodiments, the antiemetic agent is selected from tropisetron, palonosetron, ramosetron, granisetron, ondansetron, dolasetron, metoclopramide, domperidone, olanzapine, droperidol, haloperidol, chlorpromazine, prochlorperazine, alizapride, prochlorperazine, metoclopramide, casopitant, aprepitant, cyclizine, diphenhydramine, dimenhydrinate, doxylamine, meclizine, promethazine, hydroxyzine, dronabinol, sativex, midazolam, lorazepam, hyoscine, trimethobenzamide, emetrol, propofol and muscimol.

In some embodiments, provided herein is a composition for use in a method for treating chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the fosaprepitant, or a pharmaceutically acceptable salt thereof, aprepitant and the human serum albumin as described herein, and a therapeutically effective amount of at least one antiemetic agent as described herein.

In some embodiments, provided herein is a composition for use in a method for preventing chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the fosaprepitant, or a pharmaceutically acceptable salt thereof, aprepitant and the human serum albumin as described herein, and a therapeutically effective amount of at least one antiemetic agent as described herein.

In some embodiments, a pharmaceutical composition comprising the composition comprising the fosaprepitant, or a pharmaceutically acceptable salt thereof, aprepitant and the human serum albumin as described herein and an antiemetic agent are administered simultaneously.

In some embodiments, a pharmaceutical composition comprising the composition comprising the fosaprepitant, or a pharmaceutically acceptable salt thereof, aprepitant and the human serum albumin as described herein and an antiemetic agent are administered consecutively.

Also, provided herein is a pharmaceutical composition comprising the composition comprising the fosaprepitant, or a pharmaceutically acceptable salt thereof, aprepitant and the human serum albumin as described herein, at least one antiemetic agent as described herein, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, a 5-$HT_3$ antagonist (e.g., tropisetron, palonosetron, ramosetron, granisetron, ondansetron, dolasetron, or metoclopramide), and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, 0.5mg of palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, 0.25mg of palonosetron, and a pharmaceutically acceptable carrier.

In some embodiments, provided herein is a pharmaceutical composition comprising the composition the fosaprepitant, or a pharmaceutically acceptable salt thereof, aprepitant and the human serum albumin as described herein, 0.28mg of palonosetron hydrochloride, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, about 0.25mg of palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, about 0.28mg of palonosetron hydrochloride, and a pharmaceutically acceptable carrier.

In some embodiments, provided herein is a liquid pharmaceutical formulation for injection comprising the composition comprising the fosaprepitant, or a pharmaceutically acceptable salt thereof, aprepitant and the human serum albumin as described herein, 0.25mg of palonosetron, and a pharmaceutically acceptable carrier.

In some embodiments, provided herein is a liquid pharmaceutical formulation for injection comprising the composition comprising the fosaprepitant, or a pharmaceutically acceptable salt thereof, aprepitant and the human serum albumin as described herein, 0.28mg of palonosetron hydrochloride, and a pharmaceutically acceptable carrier.

In some embodiments, the liquid pharmaceutical formulation for injection is an aqueous solution. In some embodiments, the liquid pharmaceutical formulation for injection is free of solvent other than water. In some embodiments, the liquid pharmaceutical formulation for injection is substantially free of solvent other than water. In some embodiments, the liquid pharmaceutical formulation for injection is a formulation for infusion (e.g., intravenous infusion).

In some embodiments, the liquid pharmaceutical formulation for injection has pH value from about 5 to about 8. In some embodiments, the liquid pharmaceutical formulation for injection has pH value from about 5.5 to about 7.8. In some embodiments, the liquid pharmaceutical formulation for injection has pH value from about 6 to about 7.5. In some embodiments, the liquid pharmaceutical formulation for injection has pH value from about 6.5 to about 7.5. In some embodiments, the liquid pharmaceutical formulation for injection has pH value from about 6 to about 6.5. In some embodiments, the liquid pharmaceutical formulation for injection has pH value from about 6.5 to about 7. In some embodiments, the liquid liquid pharmaceutical formulation for injection has pH value from about 7 to about 7.5. In some embodiments, the liquid pharmaceutical formulation for injection has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical formulation for injection is substantially free of solvent other than water. In some embodiments, the liquid pharmaceutical formulation for injection is free of solvent other than water.

In some embodiments, provided herein is a pharmaceutical composition comprising the composition comprising the fosaprepitant, or a pharmaceutically acceptable salt thereof, aprepitant and the human serum albumin as described herein, granisetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, ondansetron, and a pharmaceutically acceptable carrier.

The methods described herein may be performed alone or in conjunction with another therapy, such as surgery, radiation, chemotherapy, immunotherapy, gene therapy, and the like.

In some embodiments, the amount of fosaprepitant, or a pharmaceutically acceptable salt thereof, that is administered to a subject in need thereof with any one of pharmaceutical compositions described herein is from about 50 mg to about 250 mg, from about 60 mg to about 240 mg, from about 70 mg to 230 mg, from about 80 mg to about 220 mg, from about 90 mg to about 210 mg, from about 100 mg to about 200 mg, or from about 125 mg to about 175 mg. In some embodiments, the amount of fosaprepitant, or a pharmaceutically acceptable salt thereof, that is administered to a subject in need thereof with any one of pharmaceutical compositions described herein is about 100 mg, about 125 mg, about 150 mg, about 175 mg, or about 200 mg. In some embodiments, the amount of fosaprepitant, or a pharmaceutically acceptable salt thereof, that is administered to a subject in need thereof with any one of pharmaceutical compositions described herein is about 150 mg. In some embodiments, the amount of fosaprepitant, or a pharmaceutically acceptable salt thereof, is administered as an intravenous infusion over 20 to 30 minutes approximately 30 minutes prior to chemotherapy.

As will be understood by those of ordinary skill in the art, the appropriate doses of fosaprepitant will be approximately those already employed in clinical therapies wherein fosaprepitant is administered alone or in combination with

other therapeutic agents. Variation in dosage will likely occur depending on the condition being treated. Appropriate effective doses will also vary, as recognized by those skilled in the art, depending on the severity of the disease, the route of administration, the sex, age and general health condition of the subject, excipient usage, the possibility of co-usage with other therapeutic treatments such as use of other agents, and the judgment of the treating physician. For example, guidance for selecting an effective dose can be determined by reference to the prescribing information for fosaprepitant.

Also, provided herein is a liquid pharmaceutical composition comprising the composition comprising the fosaprepitant, or a pharmaceutically acceptable salt thereof, the aprepitant and the human serum albumin as described herein, and a pharmaceutically acceptable carrier.

In some embodiments, provided herein is a liquid pharmaceutical composition comprising the composition comprising the fosaprepitant, or a pharmaceutically acceptable salt thereof, the aprepitant and the human serum albumin as described herein, palonosetron, and a pharmaceutically acceptable carrier.

In some embodiments, the liquid pharmaceutical composition comprises 0.25mg of palonosetron.

In some embodiments, the liquid pharmaceutical composition comprises 0.28mg of palonosetron hydrochloride.

In some embodiments, the liquid pharmaceutical composition is a reconstituted solution, reconstituted from the solid composition comprising the fosaprepitant, or a pharmaceutically acceptable salt thereof, the aprepitant and the human serum albumin as described herein.

In some embodiments, the liquid pharmaceutical composition is a reconstituted solution, reconstituted from the solid composition comprising the fosaprepitant, or a pharmaceutically acceptable salt thereof, the aprepitant and the human serum albumin as described herein and palonosetron.

In some embodiments, the liquid pharmaceutical composition is an aqueous solution. In some embodiments, the liquid pharmaceutical composition is an aqueous solution substantially free of solvent other than water. In some embodiments, the liquid pharmaceutical composition is an aqueous solution free of solvent other than water.

In some embodiments, the liquid pharmaceutical composition has pH value from about 5 to about 8. In some embodiments, the liquid pharmaceutical composition has pH value from about 5.5 to about 7.8. In some embodiments, the liquid pharmaceutical composition has pH value from about 6 to about 7.5. In some embodiments, the liquid pharmaceutical composition has pH value from about 6.5 to about 7.5. In some embodiments, the liquid pharmaceutical composition has pH value from about 6 to about 6.5. In some embodiments, the liquid pharmaceutical composition has pH value from about 6.5 to about 7. In some embodiments, the liquid pharmaceutical composition has pH value from about 7 to about 7.5. In some embodiments, the liquid pharmaceutical composition has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical composition is substantially free of solvent other than water. In some embodiments, the liquid pharmaceutical composition is free of solvent other than water.

In some embodiments, the liquid pharmaceutical composition is an aqueous reconstituted solution, reconstituted in a parenterally acceptable aqueous pharmaceutical diluent. In some embodiments, the liquid pharmaceutical composition is an aqueous reconstituted solution, reconstituted in an aqueous infusion fluid.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a parenterally acceptable aqueous pharmaceutical diluents comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution comprising a commercially available solution of human

serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution prepared from diluting a commercially available solution of human serum albumin USP for infusion with saline.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution prepared from diluting a commercially available solution of human serum albumin USP for infusion with saline.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution prepared from diluting a commercially available solution of human serum albumin USP for infusion with water.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution prepared from diluting a commercially available solution of human serum albumin USP for infusion with water.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution of human serum albumin, in which the concentration in weight of human serum albumin in the solution is in the range from 0.1% to 25% (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution of human serum albumin, in which the concentration in weight of human serum albumin in the solution is in the range from 0.1% to 25% (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 5% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 5% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 10% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 10% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 20% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 20% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 25% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 25% solution of human serum albumin USP for infusion (w/v).

Fosaprepitant easily degrades to aprepitant unless stored at low temperature. Degradation is enhanced by the presence of water. In some embodiments, all or part of aprepitant in the solid composition is degraded from fosaprepitant, or a pharmaceutically acceptable salt thereof. In some embodiments, all aprepitant in the solid composition is degraded

from fosaprepitant, or a pharmaceutically acceptable salt thereof. In some embodiments, part of aprepitant in the solid composition is degraded from fosaprepitant, or a pharmaceutically acceptable salt thereof.

In some embodiments, the liquid pharmaceutical composition contains from about 100 mg to about 150 mg of the fosaprepitant, or a pharmaceutically acceptable salt thereof. In some embodiments, the liquid pharmaceutical composition contains from about 145 mg to about 150 mg of the fosaprepitant, or a pharmaceutically acceptable salt thereof. In some embodiments, the liquid pharmaceutical composition contains about 150 mg of the fosaprepitant, or a pharmaceutically acceptable salt thereof. In some embodiments, the liquid pharmaceutical composition contains 150 mg of the fosaprepitant, or a pharmaceutically acceptable salt thereof. In some embodiments, the liquid pharmaceutical composition contains 245.3 mg of the fosaprepitant dimeglumine equivalent to 150 mg of fosaprepitant free acid.

In some embodiments, the fosaprepitant, or a pharmaceutically acceptable salt thereof, and the human serum albumin in the liquid pharmaceutical composition have a ratio by weight from about 1:1 to about 1:150. In some embodiments, the fosaprepitant, or a pharmaceutically acceptable salt thereof, and the human serum albumin in the liquid pharmaceutical composition have a ratio by weight from about 1:2 to about 1:100. In some embodiments, the fosaprepitant, or a pharmaceutically acceptable salt thereof, and the human serum albumin in the liquid pharmaceutical composition have a ratio by weight from about 1:2 to about 1:80. In some embodiments, the fosaprepitant, or a pharmaceutically acceptable salt thereof, and the human serum albumin in the liquid pharmaceutical composition have a ratio by weight from about 1:2 to about 1:50. In some embodiments, the fosaprepitant, or a pharmaceutically acceptable salt thereof, and the human serum albumin in the liquid pharmaceutical composition have a ratio by weight from about 1:2.5 to about 1:30. In some embodiments, the fosaprepitant, or a pharmaceutically acceptable salt thereof, and the human serum albumin in the liquid pharmaceutical composition have a ratio by weight from about 1:3 to about 1:20. In some embodiments, the fosaprepitant, or a pharmaceutically acceptable salt thereof, and the human serum albumin in the liquid pharmaceutical composition have a ratio by weight from about 1:3.5 to about 1:15. In some embodiments, the fosaprepitant, or a pharmaceutically acceptable salt thereof, and the human serum albumin in the liquid pharmaceutical composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the liquid pharmaceutical composition is a clear aqueous solution. In some embodiments, the liquid pharmaceutical composition is a clear aqueous solution for at least 1 hour. In some embodiments, the liquid pharmaceutical composition is a clear aqueous solution for at least 2 hours. In some embodiments, the liquid pharmaceutical composition is a clear aqueous solution for at least 3 hours. In some embodiments, the liquid pharmaceutical composition is a clear aqueous solution for at least 4 hours. In some embodiments, the liquid pharmaceutical composition is a clear aqueous solution for at least 5 hours. In some embodiments, the liquid pharmaceutical composition is a clear aqueous solution for at least 6 hours. In some embodiments, the liquid pharmaceutical composition is a clear aqueous solution for at least 24 hours.

In some embodiments, the liquid pharmaceutical composition is an injectable pharmaceutical formulation.

In some embodiments, a unit dosage form for injection comprising the liquid pharmaceutical composition contains about 150mg of the fosaprepitant. In some embodiments, a unit dosage form for injection comprising the liquid pharmaceutical composition contains 150mg of the fosaprepitant. In some embodiments, a unit dosage form for injection comprising the liquid pharmaceutical composition contains 245.3 mg of the fosaprepitant dimeglumine equivalent to 150 mg of the fosaprepitant (fosaprepitant free acid).

In some embodiments, a unit dosage form for injection comprising the liquid pharmaceutical composition contains about 150mg of fosaprepitant and 0.25mg of palonosetron. In some embodiments, a unit dosage form for injection comprising the liquid pharmaceutical composition contains 150mg of fosaprepitant and 0.25mg of palonosetron.In some embodiments, a unit dosage form for injection comprising the liquid pharmaceutical composition contains 245.3 mg of fosaprepitant dimeglumine and 0.28mg of palonosetron hydrochloride.

In some embodiments, the injectable pharmaceutical formulation is free of solvent other than water. In some embodiments, the injectable pharmaceutical formulation is substantially free of solvent other than water.

In some embodiments, the injectable pharmaceutical formulation has pH value from about 5 to about 8. In some embodiments, the injectable pharmaceutical formulation has pH value from about 5.5 to about 7.8. In some embodiments, the injectable pharmaceutical formulation has pH value from about 6 to about 7.5. In some embodiments, the injectable pharmaceutical formulation has pH value from about 6.5 to about 7.5. In some embodiments, the injectable pharmaceutical formulation has pH value from about 6 to about 6.5. In some embodiments, the injectable pharmaceutical formulation has pH value from about 6.5 to about 7. In some embodiments, the injectable pharmaceutical formulation has pH value from about 7 to about 7.5. In some embodiments, the injectable pharmaceutical formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the injectable pharmaceutical formulation is substantially free of solvent other than water. In some embodiments, the injectable pharmaceutical formulation is free of solvent other than water.

In some embodiments, the injectable pharmaceutical formulation is a reconstituted solution, reconstituted from the composition comprising the fosaprepitant, or a pharmaceutically acceptable salt thereof, the aprepitant and the human serum albumin as described herein. In some embodiments, the injectable pharmaceutical formulation is a reconstituted solution, reconstituted in an aqueous infusion fluid. In some embodiments, the aqueous infusion fluid is normal saline. In some embodiments, the aqueous infusion fluid is a dextrose solution.

In some embodiments, the injectable pharmaceutical formulation is a clear aqueous solution. In some embodiments, the injectable pharmaceutical formulation is a clear aqueous solution for at least 1 hour. In some embodiments, the injectable pharmaceutical formulation is a clear aqueous solution for at least 2 hours. In some embodiments, the injectable pharmaceutical formulation is a clear aqueous solution for at least 3 hours. In some embodiments, the injectable pharmaceutical formulation is a clear aqueous solution for at least 4 hours. In some embodiments, the injectable pharmaceutical formulation is a clear aqueous solution for at least 5 hours. In some embodiments, the injectable pharmaceutical formulation is a clear aqueous solution for at least 6 hours.

In some embodiments, the injectable pharmaceutical formulation is a clear aqueous solution for at least 1 hour at a temperature from about 0 °C to about 10 °C. In some embodiments, the injectable pharmaceutical formulation is a clear aqueous solution for at least 2 hours at a temperature from about 0 °C to about 10 °C. In some embodiments, the injectable pharmaceutical formulation is a clear aqueous solution for at least 3 hours at a temperature from about 0 °C to about 10 °C. In some embodiments, the injectable pharmaceutical formulation is a clear aqueous solution for at least 6 hours at a temperature from about 0 °C to about 10 °C. In some embodiments, the injectable pharmaceutical formulation is a clear aqueous solution for at least 8 hours at a temperature from about 0 °C to about 10 °C. In some embodiments, the injectable pharmaceutical formulation is a clear aqueous solution for at least 24 hours at a temperature from about 0 °C to about 10 °C.

Also, provided herein is a kit comprising a solid composition comprising the fosaprepitant, or a pharmaceutically acceptable salt thereof, and the aprepitant , and an aqueous solution of human serum albumin.

In some embodiments, the aqueous solution of human serum albumin in the kit has concentration of human serum albumin in the range from 0.1% to 25% (w/v).

In some embodiments, the solid composition in the kit comprises 245.3mg of fosaprepitant dimeglumine equivalent to 150 mg of fosaprepitant free acid.

In some embodiments, the solid composition in the kit comprises about 245.3mg of fosaprepitant dimeglumine.

In some embodiments, the kit comprises the solid composition comprising 245.3mg of fosaprepitant dimeglumine equivalent to 150 mg of fosaprepitant free acid and an aqueous solution of human serum albumin with the human serum albumin in the range from 0.1% to 25% (w/v).

## Methods of making

Also, provided herein are several methods to prepare a composition comprising fosaprepitant, or a pharmaceutically acceptable salt thereof, and human serum albumin as described herein, a composition comprising aprepitant and human serum albumin as described herein, or a composition comprising fosaprepitant, or a pharmaceutically acceptable salt thereof, aprepitant and human serum albumin as described herein.

### I. Compositions comprising fosaprepitant and aprepitant

In some embodiments, the present disclosure provides a method of preparing a composition comprising fosaprepitant, or a pharmaceutically acceptable salt thereof, aprepitant, and human serum albumin as described herein.

In some embodiments, the method of preparing a composition comprising fosaprepitant, or a pharmaceutically acceptable salt thereof, aprepitant and human serum albumin as described herein comprise the steps of:

(i) obtaining an organic solution comprising fosaprepitant, or a pharmaceutically acceptable salt thereof, and aprepitant in polar water- miscible organic solvent;
(ii) obtaining a first aqueous solution comprising human serum albumin (HSA); and
(iii) mixing the organic solution of step (i) and the first aqueous solution of step (ii) to obtain a second aqueous solution comprising the composition comprising fosaprepitant, aprepitant and human serum albumin.

In some embodiments, the fosaprepitant, or a pharmaceutically acceptable salt thereof, is fosaprepitant dimeglumine.

In some embodiments, the composition is solid.

In some embodiments, the composition comprises a non-covalently bond complex comprising fosaprepitant, aprepitant and the human serum albumin.

In some embodiments, the mixing step is followed by removal of solvents from the second aqueous solution

to yield the solid composition comprising fosaprepitant, aprepitant and human serum albumin. In some embodiments, the removal of aqueous solvent is conducted by rotary evaporation. In some embodiments, the removal of aqueous solvent is conducted by lyophilization.

In some embodiments, the weight ratio of fosaprepitant, or a pharmaceutically acceptable salt thereof, to aprepitant in the composition is from about 200:1 to about 1:1, from about 150:1 to about 10:1, or from about 100:1 to about 30:1. In some embodiments, the weight ratio of fosaprepitant, or a pharmaceutically acceptable salt thereof, to aprepitant in the composition is about 200:1, about 100:1, about 100:2, about 100:3, about 100:4 or about 100:5.

In some embodiments, the molar ratio of fosaprepitant, or a pharmaceutically acceptable salt thereof, to aprepitant in the composition is from about 200:1 to about 5:1, from about 150 to about 10:1, from about 100:1 to about 20:1, or from about 90:1 to about 30:1.

In some embodiments, the weight ratio of aprepitant to human serum albumin in the composition is from about 1:1 to about 1:200, from about 1:5 to about 1:150, or from about 1:10 to about 1:100. In some embodiments, the weight ratio of aprepitant to human serum albumin in the composition is about 1:5, about 1:10, about 1:13, about 1:15, about 1:20, about 1:30, about 1:33, about 1:40, about 1:50, about 1:75, or about 1:100.

In some embodiments, the fosaprepitant, or the pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2.5 to about 1:30, from about 1:3 to about 1:20, from about 1:1 to about 3:1, or from about 1:1 to about 2.5:1. In some embodiments, the weight ratio of fosaprepitant to the human serum albumin in the composition is about 1:1, about 1.5:1, about 2:1, about 2.5:1, or about 3:1. In some embodiments, the fosaprepitant and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the present disclosure provides a composition comprising fosaprepitant, or a pharmaceutically acceptable salt thereof, aprepitant, and human serum albumin prepared by any one of the processes described herein.

A non-limiting embodiments of the method are as follows.

*Formation of the organic solution*

As used herein, the term "organic solution" refers to a solution wherein at least one solvent is a non-aqueous solvent and the weight % of the non-aqueous solvent in the mixture of solvents is at least 50%, at least 60%, at least 70%, at least 90%, at least 95%, or at least 99%. In some embodiments, organic solution is a solution in which does not comprise water as a solvent.

In some embodiments, the terms "organic solvent" and "non-aqueous solvent" are used interchangeably and refer to a liquid comprising is at least 50%, at least 60%, at least 70%, at least 90%, at least 95%, or at least 99% of a solvent other than water. In some embodiments, the organic solvent does not comprise water.

In some embodiments, fosaprepitant, or a pharmaceutically acceptable salt thereof, and aprepitant are dissolved in a polar organic solvent (e.g., an alcohol such as methanol, ethanol, isopropanol, and/or n-butanol; THF, $CH_3CN$; DMF; or mixtures thereof) to form an organic solution. The polar organic solvent is miscible in water. In some embodiments, the polar organic solvent is an alcohol. In some embodiments, the polar organic solvent is ethanol or methanol, or mixtures thereof. For example, the polar organic solvent can be ethanol. In some embodiments, the polar organic solvent is methanol.

In some embodiments, the amount of polar organic solvent (e.g., methanol) is from about 0.005 mL to about 5 mL per 1 mg of solid mixture of aprepitant and fosaprepitant, or a pharmaceutically acceptable salt thereof. In some embodiments, the amount of polar organic solvent is from about 0.01 mL to about 3 mL per 1 mg of solid mixture of aprepitant and fosaprepitant, or a pharmaceutically acceptable salt thereof. In some embodiments, the amount of polar organic solvent is from about 0.01 mL to about 2 mL per 1 mg of solid mixture of aprepitant and fosaprepitant, or a pharmaceutically acceptable salt thereof. In some embodiments, the amount of polar organic solvent is from about 0.01 mL to about 1 mL per 1 mg of solid mixture of aprepitant and fosaprepitant, or a pharmaceutically acceptable salt thereof. In some embodiments, the amount of polar organic solvent is about 0.01 mL, about 0.011 mL, about 0.012 mL, about 0.013 mL, about 0.015 mL, or about 0.02 mL per 1 mg of solid mixture of aprepitant and fosaprepitant, or a pharmaceutically acceptable salt thereof

*Formation of the first aqueous solution*

In some embodiments, a defined amount of human serum albumin is dissolved in an amount of aqueous solvent

to form a first aqueous solution. In some aspects of these embodiments, the aqueous solvent is water. In some aspects of these embodiments, the aqueous solvent is saline.

In some embodiments, the amount of the aqueous solvent in the first aqueous solution is from about 0.001mL to about 10 mL per 1 mg of HSA. In some embodiments, the amount of the aqueous solvent in the first aqueous solution per 1 mg of HSA is from about 0.004mL to about 5 mL, from about 0.005 mL to about 1 mL, from about 0.008 mL to about 0.5 mL, 0.01 mL to about 0.2 mL, or from 0.01 mL to about 0.15 mL. In some embodiments, the amount of the aqueous solvent in the first aqueous solution per 1 mg of HSA is from about 0.0005 mL to about 0.025 mL, from about 0.0005 mL to about 1 mL, from about 0.0005 mL to about 0.05 mL, or from about 0.005 mL to about 0.05 mL. In some embodiments, the amount of the aqueous solvent in the first aqueous solution per 1 mg of HSA is about 0.0005 mL, about 0.005 mL, about 0.01 mL, about 0.02 mL, or about 0.025 mL.

In some embodiments, a commercially available solution of human serum albumin USP for infusion can be used to form a first aqueous solution.

In some embodiments, a commercially available solution of human serum albumin USP for infusion can be used to form a first aqueous solution when diluted with water.

In some embodiments, a commercially available solution of human serum albumin USP for infusion can be used to form a first aqueous solution when diluted with saline.

5%, 20%, and 25% solution of human serum albumin (w/v) USP for infusion are commercially available.

In some embodiments, a solution of human serum albumin USP for infusion with or without dilution to form a first aqueous solution has the concentration in weight of human serum albumin in the range from about 0.1% to about 25% (w/v).

In some embodiments, a solution of human serum albumin USP for infusion with or without dilution to form a first aqueous solution has the concentration in weight of human serum albumin in the range from about 1% to about 20% (w/v).

In some embodiments, a solution of human serum albumin USP for infusion with or without dilution to form a first aqueous solution has the concentration in weight of human serum albumin (w/v) in about 1%, 2%, 3%, 4%, 5%, 6%, 7%, 8%, 9%, 10%, 11%, 12%, 13%, 14%, 15%, 16%, 17%, 18%, 19%, or 20%.

In some embodiments, a 10% solution of human serum albumin (w/v) USP for infusion is used to form a first aqueous solution.

In some embodiments, a 5% solution of human serum albumin (w/v) USP for infusion is used to form a first aqueous solution.

In some embodiments, a 12.5% solution of human serum albumin (w/v) USP for infusion is used to form a first aqueous solution.

In some embodiments, the preparation of the organic solution and the preparation of the first aqueous solution are performed concurrently. In some embodiments, the preparation of the organic solution and the preparation of the first aqueous solution are performed sequentially. In some embodiments, the preparation of the organic solution is performed before the preparation of the first aqueous solution. In some embodiments, the preparation of the first aqueous solution is performed before the preparation of the organic solution.

*Formation of the second aqueous solution*

In some embodiments, the first aqueous solution of human serum albumin is mixed with a solid composition comprising fosaprepitant and aprepitant to form a second aqueous solution. In some embodiments, the first aqueous solution of human serum albumin is mixed with a solid composition comprising fosaprepitant dimeglumine and aprepitant to form a second aqueous solution. In some embodiments, the second aqueous solution is a clear aqueous solution.

In some embodiments, the aprepitant and the fosaprepitant in the said solid composition comprising the fosaprepitant and the aprepitant have a ratio by weight no more than 1:20. In some embodiments, the aprepitant and the fosaprepitant in the said solid composition comprising the fosaprepitant and the aprepitant have a ratio by weight no more than 1:25. In some embodiments, the aprepitant and the fosaprepitant in the said solid composition comprising the fosaprepitant and the aprepitant have a ratio by weight no more than 3:100. In some embodiments, the aprepitant and the fosaprepitant in the said solid composition comprising the fosaprepitant and the aprepitant have a ratio by weight no more than 1:50. In some embodiments, the aprepitant and the fosaprepitant in the said solid composition comprising the fosaprepitant and the aprepitant have a ratio by weight no more than 1:100. In some embodiments, the aprepitant and the fosaprepitant in the said solid composition comprising the fosaprepitant and the aprepitant have a ratio by weight no more than 1:200. In some embodiments, the aprepitant and the fosaprepitant in the said solid composition comprising the fosaprepitant and the aprepitant have a ratio by weight no more than 1:500.

In some embodiments, the fosaprepitant and the human serum albumin in the second aqueous solution have a ratio by weight from about 1:0.5 to about 1:300. In some embodiments, the fosaprepitant and the human serum albumin in the second aqueous solution have a ratio by weight from about 1:0.8 to about 1:200. In some embodiments, the

fosaprepitant and the human serum albumin in the second aqueous solution have a ratio by weight from about 1:1 to about 1:150. In some embodiments, the fosaprepitant and the human serum albumin in the second aqueous solution have a ratio by weight from about 1:2 to about 1:100. In some embodiments, the fosaprepitant and the human serum albumin in the second aqueous solution have a ratio by weight from about 1:2 to about 1:80. In some embodiments, the fosaprepitant and the human serum albumin in the second aqueous solution have a ratio by weight from about 1:2 to about 1:50. In some embodiments, the fosaprepitant and the human serum albumin in the second aqueous solution have a ratio by weight from about 1:2.5 to about 1:30. In some embodiments, the fosaprepitant and the human serum albumin in the second aqueous solution have a ratio by weight from about 1:3 to about 1:20. In some embodiments, the fosaprepitant and the human serum albumin in the second aqueous solution have a ratio by weight from about 1:3.5 to about 1:15. In some embodiments, the fosaprepitant and the human serum albumin in the second aqueous solution have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 70.

In some embodiments, the solid composition is added to the first aqueous solution to form a second aqueous solution. In some embodiments, the first aqueous solution is added to the solid composition to form a second aqueous solution. In some embodiments, the mixing is performed with agitation. In some embodiments, the mixing is performed with stirring. In some embodiments, the mixing is performed with shaking.

In some embodiments, the second aqueous can be further diluted by saline to a desired concentration for infusion.

In some embodiments, organic solution comprising fosaprepitant, or a pharmaceutically acceptable salt thereof, and aprepitant is mixed with the first aqueous solution of human serum albumin to form a second aqueous solution. In some embodiments, the second aqueous solution is a clear aqueous solution. In some embodiments, the mixing is carried out in a reaction vessel comprising aqueous solvent (e.g., water), and the volume ratio of the aqueous solvent in the reaction vessel to the volume ration of the aqueous solvent in the first aqueous solution is from about 4 to about 10.

In some embodiments, the volume ratio of the amount of the aqueous solvent (e.g., water or saline) to the amount of the polar organic solvent in the second aqueous solution is in a range from about 50:1 to about 1:50, from about 25:1 to about 1:1, from about 20:1 to about 1:1, from about 15:1 to about 1:1, from about 10:1 to about 1:1, from about 5:1 to about 1:1, from about 2.5:1 to about 1:1, from about 2.2:1 to about 1:1, from about 2:1 to about 1:1, from about 1.8:1 to about 1:1, from about 1.7:1 to about 1:1, or from about 1.5:1 to about 1:1. In some embodiments, the volume ratio of the amount of the aqueous solvent (e.g., water or saline) to the amount of the polar organic solvent in the second aqueous solution is about 1.5:1, about 1.7:1, about 1.8:1, about 2:1, about 2.2:1, or about 2.5:1.

In some embodiments, the mixing is carried out by adding the organic solution to the first aqueous solution to form the second aqueous solution. In some embodiments, the mixing is carried out by adding the organic solution to the reaction vessel comprising aqueous solvent and the first aqueous solution to form the second aqueous solution. In some embodiments, the mixing is varied out by adding the first aqueous solution to the organic solution to form the second aqueous solution. In some embodiments, the addition is dropwise. In some embodiments, the mixing is performed with agitation, stirring or shaking. In some embodiments, the mixing is carried out at the temperature from about 0 °C to about 30 °C. In some embodiments, the mixing is carried out at the temperature from about 0 °C to about 20 °C. In some embodiments, the mixing is carried out at the temperature from about 0 °C to about 10 °C. In some embodiments, the mixing is carried out at the temperature from about 0 °C to about 5 °C. In some embodiments, the mixing is carried out at the temperature about 0 °C. In some embodiments, the mixing is carried out at the temperature about 5 °C. In some embodiments, the mixing is carried out at the temperature about 10 °C.

In some embodiments, the time of mixing is in a range from about 0.1 min to about 24 hours. In some embodiments, the time of mixing is in a range from about 1 min to about 2 hour. In some embodiments, the time of mixing is in a range from about 1 min to about 1 hour. In some embodiments, the time of mixing is in a range from about 5 min to about 30 min.

*Optionally removal of water from the second aqueous solution*

In some embodiments, the water can be removed from the second aqueous solution to provide a solid composition.

In some embodiments, the second aqueous solution is filtered before removal of water. For example, the second aqueous solution can be filtered by a 0.22 micron filter before removal of water.

As used herein, the term "micron" refers to a unit of measure of one one-thousandth of a millimeter.

In some embodiments, the water is removed under a vacuum. In some embodiments, the water is removed using rotary evaporation. In some embodiments, the water is removed by lyophilization.

*Optionally reconstitution of the solid compostion*

In some embodiments the solid composition comprising the fosaprepitant, the aprepitant, and the human serum albumin is mixed with a water solution. In some embodiments, the water solution is a saline solution. In some embodiments, the water solution is a 5% Dextrose water solution. In some embodiments, the mixing is the addition of the water solution to the solid. In some embodiments, the mixing is the addition of the solid to the water solution. In some embodiments, the mixing reconstitutes the solid. In some embodiments, the mixing yields a clear aqueous solution.

Unless otherwise defined, all technical and scientific terms used herein have the same meaning as commonly understood by one of ordinary skill in the art to which this disclosure belongs. Methods and materials are described herein for use in the present disclosure; other suitable methods and materials known in the art can also be used. The materials, methods, and examples are illustrative only and not intended to be limiting. In case of conflict between the present specification and a reference mentioned herein, the present specification, including definitions, will control.

## EXAMPLES

### Materials and methods

**HPLC analysis**: The HPLC system used herein is a SHIMADZU LC-10AT vp series system, which consists of a SHIMADZU LC-10AT vp pump, a manual injector, a SHIMADZU CTO-10AS vp column oven, a SHIMADZU SPD-10A vp wavelength detector, and a SHIMADZU LC solution workstation. Agilent Zorbax XDB-C18 column (4.6 mm x 50 mm, 5 $\mu$m) is used as an analytical HPLC column. Mobile phases A and B consist of water with 0.1% trifluoroacetic acid (TFA) and methanol with 0.1% TFA, respectively. The mobile phases were delivered at a programmed linear gradient. Separation was pumped at a flow rate of 1 ml/minute, and initiated and maintained at a mobile phase ratio of 85:15 (A:B) for 1minute. The ratio was changed to 10:90 (A:B) over a period of 2 minutes using a linear curve and then maintained at 10:90 (A:B) over a period of 4.5 minutes. The mobile phase was subsequently changed back to 85:15 (A:B) over a period of 1 minute and this ratio was maintained for 1.5 minutes before the next sample was injected. The effluent is detected at a wavelength of 254 nm using a UV detector. The sample injection amount is 20 $\mu$l.

### Example 1: Composition comprising fosaprepitant and human serum albumin (HSA).

The ratio by weight of fosaprepitant dimeglumine to HSA prepared was about 1:40.

Fosaprepitant dimeglumine (10 mg) was dissolved in water (2 ml) in a vial to give a clear solution. A solution of HSA (400 mg, 2 ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 2: Composition comprising fosaprepitant and human serum albumin (HSA).

The ratio by weight of fosaprepitant dimeglumine to HSA prepared was about 1:20.

Fosaprepitant dimeglumine (10 mg) was dissolved in water (1 ml) in a vial to give a clear solution. A solution of HSA (200 mg, 1 ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 3: Composition comprising fosaprepitant and human serum albumin (HSA).

The ratio by weight of fosaprepitant dimeglumine to HSA prepared was about 1:4.

Fosaprepitant dimeglumine (25 mg) was dissolved in water (1.5 ml) in a vial to give a clear solution. A solution of HSA (100 mg, 0.5ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

**Example 4: Composition comprising fosaprepitant and human serum albumin** (HSA).

The ratio by weight of fosaprepitant dimeglumine to HSA prepared was about 1:2.

Fosaprepitant dimeglumine (50 mg) was dissolved in water (1.5 ml) in a vial to give a clear solution. A solution of HSA (100 mg, 0.5ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

**Example 5: Composition comprising fosaprepitant and human serum albumin** (HSA).

The ratio by weight of fosaprepitant dimeglumine to HSA prepared was about 1:4.

Fosaprepitant dimeglumine (50 mg) was dissolved in water (1ml) in a vial to give a clear solution. A solution of HSA (200 mg, 1ml) (20% human serum albumin solution for infusion (product name: AlbuRx) from CSL Behring) was added into the vial of the fosaprepitant dimeglumine water solution at 0 °C. Upon completion of the addition and warmed up to room temperature, a clear solution was obtained. 0.2ml of the resulting clear aqueous solution was added into a saline solution (2ml) to give a clear aqueous solution.

**Example 6: The stability of fosaprepitant in a human serum albumin (HSA) solution.**

To 3 different vials containing 25 mg of fosaprepitant dimeglumine each were added 1ml of 10% HSA solution (w/v), 1ml of 5% HSA solution (w/v), and 1ml of 2% HSA solution (w/v), respectively, at 25 °C. A clear aqueous solution was obtained initially for all 3 vials. 10% HSA solution (w/v), 5% HSA solution (w/v), and 2% HSA solution (w/v) are obtained by diluting (20% human serum albumin solution for infusion (product name: AlbuRx) with saline. The clear aqueous solutions in the 3 vials stayed clear after 1 hour, 2 hours, 3 hours, 4 hours, 6 hours, 8 hours, and 24 hours at 25 °C.

**Example 7: Composition comprising aprepitant and human serum albumin** (HSA).

The ratio by weight of aprepitant to HSA prepared was about 1:130.

Aprepitant (2 mg) was dissolved in methanol (2.6 ml) in a vial to give a clear solution. HSA (260 mg) (native fatty acid free human serum albumin purchased from SeraCare Life Sciences, product code: HS-455-80, which contains fatty acids < 0.2 mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. After 1 hour at room temperature, the solution became cloudy from precipitation.

**Example 8: Composition comprising aprepitant and human serum albumin (HSA).**

The ratio by weight of aprepitant to HSA prepared was about 1:140.

Aprepitant (2 mg) was dissolved in methanol (2.6 ml) in a vial to give a clear solution. HSA (280 mg) (native fatty acid free human serum albumin purchased from SeraCare Life Sciences, product code: HS-455-80, which contains fatty acids < 0.2 mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous

solution. After 1 hour at room temperature, the solution became cloudy from precipitation.

**Example 9: Composition comprising aprepitant and human serum albumin (HSA).**

The ratio by weight of aprepitant to HSA prepared was about 1:150.

Aprepitant (2 mg) was dissolved in methanol (2.6 ml) in a vial to give a clear solution. HSA (300 mg) (native fatty acid free human serum albumin purchased from SeraCare Life Sciences, product code: HS-455-80, which contains fatty acids < 0.2mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. The clear aqueous solution stayed clear after 1 hour at room temperature. After 1.5 hours at room temperature, the solution became cloudy from precipitation.

**Example 10: Composition comprising aprepitant and human serum albumin** (HSA).

The ratio by weight of aprepitant to HSA prepared was about 1:200.

Aprepitant (2 mg) was dissolved in methanol (3.4 ml) in a vial to give a clear solution. HSA (400 mg) (native fatty acid free human serum albumin purchased from SeraCare Life Sciences, product code: HS-455-80, which contains fatty acids < 0.2 mg/gm) as a powder was dissolved in 8 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. The clear aqueous solution stayed clear after 1.5 hours at room temperature. After 2 hours at room temperature, the solution became cloudy from precipitation.

**Example 11: Composition comprising aprepitant and human serum albumin** (HSA).

The ratio by weight of aprepitant to HSA prepared was about 1:400.

Aprepitant (2 mg) was dissolved in methanol (6.9 ml) in a vial to give a clear solution. HSA (800 mg) (native fatty acid free human serum albumin purchased from SeraCare Life Sciences, product code: HS-455-80, which contains fatty acids < 0.2 mg/gm) as a powder was dissolved in 16 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution.

**Example 12: Composition comprising aprepitant and human serum albumin** (HSA).

The ratio by weight of aprepitant to HSA prepared was about 1:220.

Aprepitant (2 mg) was dissolved in methanol (3.9 ml) in a vial to give a clear solution. HSA (440 mg) (native fatty acid free human serum albumin purchased from SeraCare Life Sciences, product code: HS-455-80, which contains fatty acids < 0.2 mg/gm) as a powder was dissolved in 9 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous

solution.

**Example 13: Composition comprising aprepitant and human serum albumin** (HSA).

The ratio by weight of aprepitant to HSA prepared was about 1:230.

Aprepitant (2 mg) was dissolved in methanol (3.9 ml) in a vial to give a clear solution. HSA (460 mg) (native fatty acid free human serum albumin purchased from SeraCare Life Sciences, product code: HS-455-80, which contains fatty acids < 0.2 mg/gm) as a powder was dissolved in 9 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.
A sample of 100 mg of the lyophilized solid was reconstituted by adding 2 mL water to give a clear aqueous solution.

**Example 14: Composition comprising aprepitant and human serum albumin (HSA).**

The ratio by weight of aprepitant to HSA prepared was about 1:250.

Aprepitant (10 mg) was dissolved in methanol (10.7 ml) in a vial to give a clear solution. HSA (2500 mg) (native fatty acid free human serum albumin purchased from
SeraCare Life Sciences, product code: HS-455-80, which contains fatty acids < 0.2 mg/gm) as a powder was dissolved in 25 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.
A sample of 100 mg of the lyophilized solid was reconstituted by adding 2 mL water to give a clear aqueous solution.

**Example 15: Measure the correlation between HPLC peak area and the aprepitant concentration.**

Methanol solutions of aprepitant in 6 different concentrations, 0.025 mg/ml, 0.0375 mg/ml, 0.05 mg/ml, 0.075 mg/ml, 0.1 mg/ml, and 0.15 mg/ml, were prepared. The 6 aprepitant methanol solutions were analyzed in HPLC. The peak area and concentration of aprepitant were correlated using linear regression. The linear regression data is shown as below.

$$Y \text{ (peak area)} = 3508 + 1.67218E6 * X \text{ (concentration)}, R = 0.99994, P < 0.0001.$$

**Example 16: Measure the aprepitant concentrations in the clear aqueous solutions before and after the filtration at 0 hour, and after the filtration at 1, 2, 3, 4, 5, and 6 hours.**

2300 mg of the lyophilized solid of the composition comprising aprepitant and HSA from Example 14 (The ratio by weight of aprepitant to HSA is about 1:250) was dissolved in 23 ml of water to give a clear aqueous solution, which was kept at about 25 °C. Immediately after the lyophilized solid was dissolved in water, 4 ml of the clear aqueous solution was taken out from the 23 ml solution. Then 1 ml of the solution was taken out from the 4 ml clear aqueous solution to give the solution AP-0-0h, and the remaining 3 ml of the solution was filtered by the same 0.22 micron aqueous phase filter at 1ml at a time to give the solutions AP-1-0h, AP-2-0h, and AP-3-0h. To 300 μl of the solutions AP-0-0h and AP-3-0h were added 700 μl of acetonitrile separately. The mixtures were vortexed for seconds and then centrifuged at 4,000 g for 5 minutes. The supernatants were removed and collected followed by injection on HPLC. The same procedure was repeated one more time for each of solutions AP-0-0h and AP-3-0h. Based on the HPLC data and the measurement data of Example 15, the aprepitant concentrations of the solutions of AP-0-0h and AP-3-0h have been calculated and shown in the Table 1. At 0 hour, the aprepitant concentration of the clear aqueous solution after the filtration was about 98.7% of the aprepitant concentration of the clear aqueous solution before the filtration.

**Table 1**

| Solution Number | Aprepitant Concentration (mg/ml) | Average aprepitant Concentration (mg/ml) |
|---|---|---|
| AP-0-0h-1 | 0.3623 | 0.3637 |
| AP-0-0h-2 | 0.3650 | |
| AP-3-0h-1 | 0.3637 | 0.3590 |
| AP-3-0h-2 | 0.3543 | |

At 1 hour, 3 ml of the clear aqueous solution was taken out from the remaining 19 ml of the aqueous solution. Then 1 ml of the solution was taken out from the 3 ml clear aqueous solution and filtered by a 0.22 micron aqueous phase filter to give the solution AP-1-1h, and the remaining 2 ml of the solution was filtered by the same 0.22 micron aqueous phase filter at 1 ml at a time to give the solutions AP-2-1h and AP-3-1h. To 300 μl of the solution AP-3-1h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-1h. Based on the HPLC data and the measurement data of Example 15, the aprepitant concentrations of the solution AP-3-1h have been calculated and shown in the Table 2. At 1 hour, the aprepitant concentration of the clear aqueous solution after the filtration was about 97.8% of the aprepitant concentration of the clear aqueous solution at 0 hour before the filtration.

**Table 2**

| Solution Number | Aprepitant Concentration (mg/ml) | Average aprepitant Concentration (mg/ml) |
|---|---|---|
| AP-3-1h -1 | 0.3563 | 0.3557 |
| AP-3-1h -2 | 0.3550 | |

At 2 hours, 3 ml of the clear aqueous solution was taken out from the remaining 16 ml of the aqueous solution. Then 1 ml of the solution was taken out from the 3 ml clear aqueous solution and filtered by a 0.22 micron aqueous phase filter to give the solution AP-1-2h, and the remaining 2 ml of the solution was filtered by the same 0.22 micron aqueous phase filter at 1 ml at a time to give the solutions AP-2-2h and AP-3-2h. To 300 μl of the solution AP-3-2h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-2h. Based on the HPLC data and the measurement data of sample 15, the aprepitant concentrations of the solution AP-3-2h have been calculated and shown in the Table 3. At 2 hours, the aprepitant concentration of the clear aqueous solution after the filtration was about 98.7% of the aprepitant concentration of the clear aqueous solution at 0 hour before the filtration.

**Table 3**

| Solution Number | Aprepitant Concentration (mg/ml) | Average aprepitant Concentration (mg/ml) |
|---|---|---|
| AP-3-2h -1 | 0.3587 | 0.3589 |
| AP-3-2h -2 | 0.3590 | |

At 3 hours, 3 ml of the clear aqueous solution was taken out from the remaining 13 ml of the aqueous solution. Then 1 ml of the solution was taken out from the 3 ml clear aqueous solution and filtered by a 0.22 micron aqueous phase filter to give the solution AP-1-3h, and the remaining 2 ml of the solution was filtered by the same 0.22 micron aqueous phase filter at 1 ml at a time to give the solutions AP-2-3h and AP-3-3h. To 300 μl of the solution AP-3-3h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-3h. Based on the HPLC data and the measurement data of sample 15, the aprepitant concentrations of the solution AP-3-3h have been calculated and shown in the Table 4. At 3 hours, the aprepitant concentration of the clear aqueous solution after the filtration was about 98.5% of the aprepitant concentration of the clear aqueous solution at 0 hour before the filtration.

Table 4

| Solution Number | Aprepitant Concentration (mg/ml) | Average aprepitant Concentration (mg/ml) |
|---|---|---|
| AP-3-3h -1 | 0.3590 | 0.3582 |
| AP-3-3h -2 | 0.3573 | |

At 4 hours, 3 ml of the clear aqueous solution was taken out from the remaining 10 ml of the aqueous solution. Then 1 ml of the solution was taken out from the 3 ml clear aqueous solution and filtered by a 0.22 micron aqueous phase filter to give the solution AP-1-4h, and the remaining 2 ml of the solution was filtered by the same 0.22 micron aqueous phase filter at 1 ml at a time to give the solutions AP-2-4h and AP-3-4h. To 300 μl of the solution AP-3-4h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-4h. Based on the HPLC data and the measurement data of sample 15, the aprepitant concentrations of the solution AP-3-4h have been calculated and shown in the Table 5. At 4 hours, the aprepitant concentration of the clear aqueous solution after the filtration was about 98.1% of the aprepitant concentration of the clear aqueous solution at 0 hour before the filtration.

**Table 5**

| Solution Number | Aprepitant Concentration (mg/ml) | Average aprepitant Concentration (mg/ml) |
|---|---|---|
| AP-3-4h -1 | 0.3573 | 0.3567 |
| AP-3-4h -2 | 0.3560 | |

At 5 hours, 3 ml of the clear aqueous solution was taken out from the remaining 7 ml of the aqueous solution. Then 1 ml of the solution was taken out from the 3 ml clear aqueous solution and filtered by a 0.22 micron aqueous phase filter to give the solution AP-1-5h, and the remaining 2 ml of the solution was filtered by the same 0.22 micron aqueous phase filter at 1 ml at a time to give the solutions AP-2-5h and AP-3-5h. To 300 μl of the solution AP-3-5h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-5h. Based on the HPLC data and the measurement data of sample 15, the aprepitant concentrations of the solution AP-3-5h have been calculated and shown in the Table 6. At 5 hours, the aprepitant concentration of the clear aqueous solution after the filtration was about 97.8% of the aprepitant concentration of the clear aqueous solution at 0 hour before the filtration.

**Table 6**

| Solution Number | Aprepitant Concentration (mg/ml) | Average aprepitant Concentration (mg/ml) |
|---|---|---|
| AP-3-5h -1 | 0.3540 | 0.3557 |
| AP-3-5h -2 | 0.3573 | |

At 6 hours, 3 ml of the clear aqueous solution was taken out from the remaining 4 ml of the aqueous solution. Then 1 ml of the solution was taken out from the 3 ml clear aqueous solution and filtered by a 0.22 micron aqueous phase filter to give the solution AP-1-6h, and the remaining 2 ml of the solution was filtered by the same 0.22 micron aqueous phase filter at 1 ml at a time to give the solutions AP-2-6h and AP-3-6h. To 300 μl of the solution AP-3-6h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-6h. Based on the HPLC data and the measurement data of sample 15, the aprepitant concentrations of the solution AP-3-6h have been calculated and shown in the Table 7. At 6 hours, the aprepitant concentration of the clear aqueous solution after the filtration was about 98.3% of the aprepitant concentration of the clear aqueous solution at 0 hour before the filtration.

**Table 7**

| Solution Number | Aprepitant Concentration (mg/ml) | Average aprepitant Concentration (mg/ml) |
|---|---|---|
| AP-3-6h -1 | 0.3577 | 0.3575 |

(continued)

| Solution Number | Aprepitant Concentration (mg/ml) | Average aprepitant Concentration (mg/ml) |
|---|---|---|
| AP-3-6h -2 | 0.3573 | |

**Example 17: Measure the stability of the solid composition comprising fosaprepitant and aprepitant in an aqueous solution with or without HSA.**

The ratio by weight of fosaprepitant dimeglumine to aprepitant in the solid composition was about 100:6.

Aprepitant (6 mg) and fosaprepitant dimeglumine (100 mg) was dissolved in 1ml of methanol to give a clear solution. Then methanol in the solution was removed to give a white solid. 20 mg each of the white solid obtained was added into each vial of the 3 vials. Water (1ml), 10% HSA solution (w/v) (1ml), 20% HSA solution (w/v) (1ml) were added into each vial with 20mg of the white solid of fosaprepitant dimeglumine and aprepitant separately. 20% HSA (w/v) solution is a 20% human serum albumin solution for infusion (product name: AlbuRx) from CSL Behring. 10% HSA solution (w/v) was obtained by diluting from the 20% HSA (w/v) solution with water. The white solids of the 3 vials were all dissolved to give a clear solution initially. The stability of the 3 solutions of fosaprepitant dimeglumine and aprepitant was shown in the table 8. In the table 8, "clear" means a clear solution, and "precipitation" means white precipitation formed in the solution.

**Table 8**

| t (minutes) | 0 | 5 | 15 | 30 |
|---|---|---|---|---|
| water (1ml) | clear | precipitation | precipitation | precipitation |
| 10% HSA (w/v) (1ml) | clear | clear | precipitation | precipitation |
| 20% HSA (w/v) (1ml) | clear | clear | clear | precipitation |

**Example 18: Measure the stability of the solid composition comprising fosaprepitant and aprepitant in an aqueous solution with or without HSA.**

The ratio by weight of fosaprepitant dimeglumine to aprepitant in the solid composition was about 100:5.

Aprepitant (5 mg) and fosaprepitant dimeglumine (100 mg) was dissolved in 1ml of methanol to give a clear solution. Then methanol in the solution was removed to give a white solid. 25 mg each of the white solid obtained was added into each vial of the 3 vials. Water (1ml), 10% HSA solution (w/v) (1ml), 20% HSA solution (w/v) (1ml) were added into each vial with 25 mg of the white solid of fosaprepitant dimeglumine and aprepitant separately. 20% has (w/v) solution is a 20% human serum albumin solution for infusion (product name: AlbuRx) from CSL Behring. 10% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 9. In the table 9, "clear" means a clear solution, and "precipitation" means white precipitation formed in the solution.

**Table 9**

| t (minutes) | 0 | 5 | 15 | 30 |
|---|---|---|---|---|
| water (1ml) | clear | precipitation | precipitation | precipitation |
| 10% HSA (w/v) (1ml) | clear | clear | clear | precipitation |
| 20% HSA (w/v) (1ml) | clear | clear | clear | precipitation |

**Example 19: Measure the stability of the solid composition comprising fosaprepitant and aprepitant in an aqueous solution with or without HSA.**

The ratio by weight of fosaprepitant dimeglumine to aprepitant in the solid composition was about 100:4.

Aprepitant (4 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 10% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 10. In the table 10, "clear" means a clear solution, and "precipitation" means white precipitation formed in the solution.

**Table 10**

| t (minutes) | 0 | 5 | 15 | 30 | 60 | 120 | 180 |
|---|---|---|---|---|---|---|---|
| water (1ml) | clear | precipitation | precipitation | precipitation | precipitation | precipitation | precipitation |
| 10% HSA (w/v) (1ml) | clear | clear | clear | clear | precipitation | precipitation | precipitation |
| 20% HSA (w/v) (1ml) | clear | clear | clear | clear | clear | precipitation | precipitation |
| 20% HSA (w/v) (2ml) | clear | clear | clear | clear | clear | clear | precipitation |

**Example 20: Measure the stability of the solid composition comprising fosaprepitant and aprepitant in an aqueous solution with or without HSA.**

The ratio by weight of fosaprepitant dimeglumine to aprepitant in the solid composition was about 100:3.

Aprepitant (3 mg) and fosaprepitant dimeglumine (100 mg) was dissolved in 1ml of methanol to give a clear solution. Then methanol in the solution was removed to give a white solid. 25 mg each of the white solid obtained was added into each vial of the 3 vials. Water (1ml), 10% HSA solution (w/v) (1ml), 20% HSA solution (w/v) (1ml) were added into each vial with 25 mg of the white solid of fosaprepitant dimeglumine and aprepitant separately. 20% HSA (w/v) solution is a 20% human serum albumin solution for infusion (product name: AlbuRx) from CSL Behring. 5% HSA solution (w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 11. In the table 11, "clear" means a clear solution, and "precipitation" means white precipitation formed in the solution.

**Table 11**

| t (minutes) | 0 | 5 | 10 | 120 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | precipitation | precipitation | precipitation |
| 10% HSA (w/v) (1ml) | clear | clear | clear | clear | clear | clear | precipitation |

**Example 21: Measure the stability of the solid composition comprising fosaprepitant and aprepitant in an aqueous solution with or without HSA.**

The ratio by weight of fosaprepitant dimeglumine to aprepitant in the solid composition was about 100:2.

Aprepitant (2 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 5% HSA solution

(w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 12. In the table 12, "clear" means a clear solution, and "precipitation" means white precipitations formed in the solution.

**Table 12**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | clear | clear | clear |
| 10% HSA (w/v) (1ml) | clear | clear | clear | clear | clear | clear | clear |

**Example 22: Measure the stability of the solid composition comprising fosaprepitant and aprepitant in an aqueous solution with or without HSA.**

The ratio by weight of fosaprepitant dimeglumine to aprepitant in the solid composition was about 100:1.

Aprepitant (1 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 5% HSA solution (w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 13. In the table 13, "clear" means a clear solution, and "precipitation" means white precipitations formed in the solution.

**Table 13**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | clear | clear | clear |
| 10% HSA (w/v) (1ml) | clear | clear | clear | clear | clear | clear | clear |

**Example 23: Measure the stability of the solid composition comprising fosaprepitant and aprepitant in an aqueous solution with or without HSA.**

The ratio by weight of fosaprepitant dimeglumine to aprepitant in the solid composition was about 100:0.5.

Aprepitant (0.5 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 2% HSA solution (w/v) and 3% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 14. In the table 14, "clear" means a clear solution, and "precipitation" means white precipitations formed in the solution.

**Table 14**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 2% HSA (w/v) (1ml) | clear | clear | clear | clear | clear | clear | precipitation |

(continued)

| t (minutes) | 0 | 5 | 10 | 60 | 180 | 300 | overnight |
|---|---|---|---|---|---|---|---|
| 3% HSA (w/v) (1ml) | clear | clear | clear | clear | clear | clear | precipitation |

**Example 24: Measure the stability of the solid composition comprising fosaprepitant and aprepitant in an aqueous solution with or without HSA.**

The ratio by weight of fosaprepitant dimeglumine to aprepitant in the solid composition was about 100:0.3.

Aprepitant (0.3 mg) and fosaprepitant dimeglumine (100 mg) was dissolved in 1 ml of methanol to give a clear solution. Then methanol in the solution was removed to give a white solid. 25 mg each of the white solid obtained was added into each vial of the 3 vials. Water (1 ml), 10% HSA solution (w/v) (1 ml), 20% HSA solution (w/v) (1 ml) were added into each vial with 25 mg of the white solid of fosaprepitant dimeglumine and aprepitant separately. 20% HSA (w/v) solution is a 20% human serum albumin solution for infusion (product name: AlbuRx) from CSL Behring. 2% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 15. In the table 15, "clear" means a clear solution, and "precipitation" means white precipitations formed in the solution.

**Table 15**

| t (minutes) | 0 | 5 | 10 | 60 | 180 | 300 | overnight |
|---|---|---|---|---|---|---|---|
| water (1ml) | clear | clear | clear | clear | clear | clear | precipitation |
| 2% HSA (w/v) (1ml) | clear | clear | clear | clear | clear | clear | clear |

**Example 25: Composition comprising fosaprepitant, aprepitant, and human serum albumin (HSA).**

The ratio by weight of fosaprepitant dimeglumine to aprepitant was about 100:1, and the ratio by weight of fosaprepitant dimeglumine to HSA prepared was about 1:1.

Fosaprepitant dimeglumine (100 mg) and aprepitant (1 mg) were dissolved in methanol (1.1 ml) in a vial to give a clear solution. A solution of HSA (100 mg, 0.5 ml) (20% human serum albumin solution for infusion (product name: AlbuRx) from CSL Behring) was added into 2 ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 16. In the table 16, "clear" means a clear solution, and "precipitation" means white precipitations formed in the solution.

**Table 16**

| t (hours) | 1 | 2 | 3 | 4 |
|---|---|---|---|---|
| water (2ml) | clear | clear | clear | clear |

**Example 26: Composition comprising fosaprepitant, aprepitant, and human serum albumin (HSA).**

The ratio by weight of fosaprepitant dimeglumine to aprepitant was about 100:2, and the ratio by weight of fosaprepitant dimeglumine to HSA prepared was about 1:1.

Fosaprepitant dimeglumine (100 mg) and aprepitant (2mg) were dissolved in methanol (1.2 ml) in a vial to give a clear solution. A solution of HSA (100 mg, 0.5ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum

to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 17. In the table 17, "clear" means a clear solution, and "precipitation" means white precipitations formed in the solution.

**Table 17**

| t (hours) | 1 | 2 | 3 | 4 |
|---|---|---|---|---|
| water (2ml) | clear | clear | precipitation | precipitation |

**Example 27: Composition comprising fosaprepitant, aprepitant, and human serum albumin (HSA).**

The ratio by weight of fosaprepitant dimeglumine to aprepitant was about 100:3, and the ratio by weight of fosaprepitant dimeglumine to HSA prepared was about 1:1.

Fosaprepitant dimeglumine (100 mg) and aprepitant (3mg) were dissolved in methanol (1.3 ml) in a vial to give a clear solution. A solution of HSA (100 mg, 0.5ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about one hour.

**Example 28: Composition comprising fosaprepitant, aprepitant, and human serum albumin (HSA).**

The ratio by weight of fosaprepitant dimeglumine to aprepitant was about 100:1, and the ratio by weight of fosaprepitant dimeglumine to HSA prepared was about 2.5:1.

Fosaprepitant dimeglumine (100 mg) and aprepitant (1mg) were dissolved in methanol (1.1 ml) in a vial to give a clear solution. A solution of HSA (40 mg, 0.2ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 18. In the table 18, "clear" means a clear solution, and "precipitation" means white precipitations formed in the solution.

**Table 18**

| t (hours) | 1 | 2 | 3 | 4 |
|---|---|---|---|---|
| water (2ml) | clear | clear | clear | clear |

**Example 29: Composition comprising fosaprepitant, aprepitant, and human serum albumin (HSA).**

The ratio by weight of fosaprepitant dimeglumine to aprepitant was about 100:2, and the ratio by weight of fosaprepitant dimeglumine to HSA prepared was about 2.5:1.

Fosaprepitant dimeglumine (100 mg) and aprepitant (2 mg) were dissolved in methanol (1.2 ml) in a vial to give a clear solution. A solution of HSA (40 mg, 0.2 ml) (20% human serum albumin solution for infusion (product name: AlbuRx) from CSL Behring) was added into 2 ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about 40

minutes.

**Example 30: Composition comprising fosaprepitant, aprepitant, and human serum albumin (HSA).**

The ratio by weight of fosaprepitant dimeglumine to aprepitant was about 100:3, and the ratio by weight of fosaprepitant dimeglumine to HSA prepared was about 2.5:1.

Fosaprepitant dimeglumine (100 mg) and aprepitant (3mg) were dissolved in methanol (1.3 ml) in a vial to give a clear solution. A solution of HSA (40 mg, 0.2ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about 30 minutes.

**Example 31: Measuring pH value of the aqueous solution comprising fosaprepitant dimeglumine with or without human serum albumin (HSA)**

24.5mg of fosaprepitant dimeglumine was dissolved in 15ml of 0.9% saline solution to give a clear aqueous solution, which was kept at about 25 °C and measured for pH value. The pH value of the clear aqueous solution is 7.70 (3 measurement points: 7.70, 7.70, and 7.70).

24.5mg of fosaprepitant dimeglumine was dissolved in 15ml of a HSA solution, which was prepared by adding 0.5ml of 20% human serum albumin solution for infusion (product name: AlbuRx) from CSL Behring into 14.5ml of 0.9% saline solution, to give a clear aqueous solution. The resulting clear aqueous solution was kept at about 25 °C and measured for pH value. The pH value of the clear aqueous solution is 7.15 (3 measurement points: 7.15, 7.15, and 7.16).

**Example 32: Measuring pH value of the aqueous solution comprising fosaprepitant dimeglumine and palonosetron hydrochloride with human serum albumin (HSA)**

245.3mg of fosaprepitant dimeglumine and 0.28mg of palonosetron hydrochloride were dissolved in methanol, and then methanol was removed from the solution to give a white solid. 24.5mg of the resulting white solid was dissolved in 15ml of a HSA solution, which was prepared by adding 0.5ml of 20% human serum albumin solution for infusion (product name: AlbuRx) from CSL Behring into 14.5ml of 0.9% saline solution, to give a clear aqueous solution. The resulting clear aqueous solution was kept at about 25 °C and measured for pH value. The pH value of the clear aqueous solution is 7.09 (3 measurement points: 7.09, 7.10, and 7.09).

## Claims

1. A composition comprising

   fosaprepitant, or a pharmaceutically acceptable salt thereof,
   aprepitant, or a pharmaceutically acceptable salt thereof, and
   human serum albumin,
   wherein the fosaprepitant, or a pharmaceutically acceptable salt thereof, and the human serum albumin in the composition have a ratio by weight from about 1:0.5 to about 1:300.

2. The composition of claim 1, wherein the aprepitant, or a pharmaceutically acceptable salt thereof, and the fosaprepitant, or a pharmaceutically acceptable salt thereof, in the composition have a ratio by weight no more than 1:100.

3. The composition of claim 1, wherein the aprepitant, or a pharmaceutically acceptable salt thereof, and the fosaprepitant, or a pharmaceutically acceptable salt thereof, in the composition have a ratio by weight no more than 1:200.

4. The composition of claim 1, wherein the fosaprepitant, or a pharmaceutically acceptable salt thereof, and the human serum albumin in the composition have a ratio by weight from about 1:1 to about 1:150.

5. A liquid pharmaceutical composition comprising the composition of claim 1-4, and a pharmaceutically acceptable carrier, wherein the liquid pharmaceutical composition is an aqueous solution substantially free of solvent other than water.

6. The liquid pharmaceutical composition of claim 5, wherein the liquid pharmaceutical composition is an aqueous reconstituted solution, prepared by reconstituting a solid composition comprising the fosaprepitant, or a pharmaceutically acceptable salt thereof, and the aprepitant, or a pharmaceutically acceptable salt thereof, in a parenterally acceptable aqueous pharmaceutical diluent comprising human serum albumin.

7. The liquid pharmaceutical composition of claim 5, wherein the liquid pharmaceutical composition is an aqueous reconstituted solution, prepared by reconstituting a solid composition comprising the fosaprepitant, or a pharmaceutically acceptable salt thereof, and the aprepitant, or a pharmaceutically acceptable salt thereof, in an aqueous solution of human serum albumin, in which the concentration in weight of human serum albumin in the solution is in the range from 0.1% to 25% (w/v).

8. The liquid pharmaceutical composition of claim 5, wherein the liquid pharmaceutical composition is a clear aqueous solution.

9. A kit comprising a solid composition comprising fosaprepitant, or a pharmaceutically acceptable salt thereof, and aprepitant, or a pharmaceutically acceptable salt thereof, and an aqueous solution of human serum albumin.

10. The kit of claim 9, wherein the solid composition in the kit comprises about 245.3 mg of fosaprepitant dimeglumine.

11. The kit of claim 9, wherein the aqueous solution of human serum albumin in the kit has concentration of human serum albumin in the range from 0.1% to 25% (w/v).

12. A kit comprising a solid composition comprising fosaprepitant, or a pharmaceutically acceptable salt thereof, and an aqueous solution of human serum albumin.

13. The kit of claim 12, wherein the solid composition in the kit comprises 245.3 mg of fosaprepitant dimeglumine equivalent to 150 mg of fosaprepitant free acid.

14. The kit of claim 12, wherein the aqueous solution of human serum albumin in the kit has concentration of human serum albumin in the range from 0.1% to 25% (w/v).

15. The kit of claim 12, wherein the solid composition comprising 245.3 mg of fosaprepitant dimeglumine equivalent to 150 mg of fosaprepitant free acid and an aqueous solution of human serum albumin with the human serum albumin concentration in the range from 0.1% to 25% (w/v).

## Patentansprüche

1. Zusammensetzung, umfassend

   Fosaprepitant oder ein pharmazeutisch verträgliches Salz davon,
   Aprepitant oder ein pharmazeutisch verträgliches Salz davon und
   menschliches Serumalbumin,
   wobei das Fosaprepitant oder ein pharmazeutisch verträgliches Salz davon und das menschliche Serumalbumin in der Zusammensetzung ein Gewichtsverhältnis von etwa 1:0,5 bis etwa 1:300 aufweisen.

2. Zusammensetzung gemäß Anspruch 1, wobei das Aprepitant oder ein pharmazeutisch verträgliches Salz davon und das Fosaprepitant oder ein pharmazeutisch verträgliches Salz davon in der Zusammensetzung ein Gewichtsverhältnis von nicht mehr als 1:100 aufweisen.

3. Zusammensetzung gemäß Anspruch 1, wobei das Aprepitant oder ein pharmazeutisch verträgliches Salz davon und das Fosaprepitant oder ein pharmazeutisch verträgliches Salz davon in der Zusammensetzung ein Gewichtsverhältnis von nicht mehr als 1:200 aufweisen.

**4.** Zusammensetzung gemäß Anspruch 1, wobei das Fosaprepitant oder ein pharmazeutisch verträgliches Salz davon und das menschliche Serumalbumin in der Zusammensetzung ein Gewichtsverhältnis von etwa 1:1 bis etwa 1:150 aufweisen.

**5.** Flüssige pharmazeutische Zusammensetzung, umfassend die Zusammensetzung gemäß Anspruch 1-4 und einen pharmazeutisch verträglichen Träger, wobei die flüssige pharmazeutische Zusammensetzung eine wässrige Lösung ist, die im Wesentlichen frei von Lösungsmittel ist, das von Wasser verschieden ist.

**6.** Flüssige pharmazeutische Zusammensetzung gemäß Anspruch 5, wobei die flüssige pharmazeutische Zusammensetzung eine wässrige rekonstituierte Lösung ist, die durch Rekonstituieren einer festen Zusammensetzung, die das Fosaprepitant oder ein pharmazeutisch verträgliches Salz davon und das Aprepitant oder ein pharmazeutisch verträgliches Salz davon umfasst, in einem parenteral verträglichen wässrigen pharmazeutischen Verdünnungsmittel, das menschliches Serumalbumin umfasst, hergestellt ist.

**7.** Flüssige pharmazeutische Zusammensetzung gemäß Anspruch 5, wobei die flüssige pharmazeutische Zusammensetzung eine wässrige rekonstituierte Lösung ist, die durch Rekonstituieren einer festen Zusammensetzung, die das Fosaprepitant oder ein pharmazeutisch verträgliches Salz davon und das Aprepitant oder ein pharmazeutisch verträgliches Salz davon umfasst, in einer wässrigen Lösung von menschlichem Serumalbumin hergestellt ist, wobei die Gewichtskonzentration von menschlichem Serumalbumin in der Lösung in dem Bereich von 0,1 % bis 25 % (Gew./Vol.) liegt.

**8.** Flüssige pharmazeutische Zusammensetzung gemäß Anspruch 5, wobei die flüssige pharmazeutische Zusammensetzung eine klare wässrige Lösung ist.

**9.** Kit, umfassend eine feste Zusammensetzung, die Fosaprepitant oder ein pharmazeutisch verträgliches Salz davon und das Aprepitant oder ein pharmazeutisch verträgliches Salz davon umfasst, und eine wässrige Lösung von menschlichem Serumalbumin.

**10.** Kit gemäß Anspruch 9, wobei die feste Zusammensetzung in dem Kit etwa 245,3 mg Fosaprepitantdimeglumin umfasst.

**11.** Kit gemäß Anspruch 9, wobei die wässrige Lösung von menschlichem Serumalbumin in dem Kit eine Konzentration von menschlichem Serumalbumin in dem Bereich von 0,1 % bis 25 % (Gew./Vol.) aufweist.

**12.** Kit, umfassend eine feste Zusammensetzung, die Fosaprepitant oder ein pharmazeutisch verträgliches Salz davon umfasst, und eine wässrige Lösung von menschlichem Serumalbumin.

**13.** Kit gemäß Anspruch 12, wobei die feste Zusammensetzung in dem Kit 245,3 mg Fosaprepitantdimeglumin, äquivalent zu 150 mg Fosaprepitant als freie Säure, umfasst.

**14.** Kit gemäß Anspruch 12, wobei die wässrige Lösung von menschlichem Serumalbumin in dem Kit eine Konzentration von menschlichem Serumalbumin in dem Bereich von 0,1 % bis 25 % (Gew./Vol.) aufweist.

**15.** Kit gemäß Anspruch 12, wobei die feste Zusammensetzung 245,3 mg Fosaprepitantdimeglumin, äquivalent zu 150 mg Fosaprepitant als freie Säure, umfasst und die wässrige Lösung von menschlichem Serumalbumin die Konzentration von menschlichem Serumalbumin in dem Bereich von 0,1 % bis 25 % (Gew./Vol.) aufweist.

## Revendications

**1.** Composition comprenant

du fosaprépitant, ou un sel pharmaceutiquement acceptable correspondant,
de l'aprépitant, ou un sel pharmaceutiquement acceptable correspondant, et
de l'albumine sérique humaine,
le fosaprépitant, ou un sel pharmaceutiquement acceptable correspondant, et l'albumine sérique humaine dans la composition possédant un rapport en poids allant d'environ 1 : 0,5 à environ 1 : 300.

**2.** Composition selon la revendication 1, l'aprépitant, ou un sel pharmaceutiquement acceptable correspondant, et le fosaprépitant, ou un sel pharmaceutiquement acceptable correspondant, dans la composition possédant un rapport en poids non supérieur à 1 : 100.

**3.** Composition selon la revendication 1, l'aprépitant, ou un sel pharmaceutiquement acceptable correspondant, et le fosaprépitant, ou un sel pharmaceutiquement acceptable correspondant, dans la composition possédant un rapport en poids non supérieur à 1 : 200.

**4.** Composition selon la revendication 1, le fosaprépitant, ou un sel pharmaceutiquement acceptable correspondant, et l'albumine sérique humaine dans la composition possédant un rapport en poids allant d'environ 1 : 1 à environ 1 : 150.

**5.** Composition pharmaceutique liquide comprenant la composition selon les revendications 1 à 4, et un support pharmaceutiquement acceptable, la composition pharmaceutique liquide étant une solution aqueuse sensiblement exempte de solvant autre que l'eau.

**6.** Composition pharmaceutique liquide selon la revendication 5, la composition pharmaceutique liquide étant une solution reconstituée aqueuse, préparée en reconstituant une composition solide comprenant le fosaprépitant, ou un sel pharmaceutiquement acceptable correspondant, et l'aprépitant, ou un sel pharmaceutiquement acceptable correspondant, dans un diluant pharmaceutique aqueux acceptable sur le plan parentéral comprenant de l'albumine sérique humaine.

**7.** Composition pharmaceutique liquide selon la revendication 5, la composition pharmaceutique liquide étant une solution reconstituée aqueuse, préparée en reconstituant une composition solide comprenant le fosaprépitant, ou un sel pharmaceutiquement acceptable correspondant, et l'aprépitant, ou un sel pharmaceutiquement acceptable correspondant, dans une solution aqueuse d'albumine sérique humaine, dans laquelle la concentration en poids d'albumine sérique humaine dans la solution est dans la plage de 0,1 % à 25 % (p/v).

**8.** Composition pharmaceutique liquide selon la revendication 5, la composition pharmaceutique liquide étant une solution aqueuse limpide.

**9.** Kit comprenant une composition solide comprenant du fosaprépitant, ou un sel pharmaceutiquement acceptable correspondant, et de l'aprépitant, ou un sel pharmaceutiquement acceptable correspondant, et une solution aqueuse d'albumine sérique humaine.

**10.** Kit selon la revendication 9, la composition solide dans le kit comprenant environ 245,3 mg de fosaprépitant diméglumine.

**11.** Kit selon la revendication 9, la solution aqueuse d'albumine sérique humaine dans le kit possédant une concentration d'albumine sérique humaine dans la plage de 0,1 % à 25 % (p/v).

**12.** Kit comprenant une composition solide comprenant du fosaprépitant, ou un sel pharmaceutiquement acceptable correspondant, et une solution aqueuse d'albumine sérique humaine.

**13.** Kit selon la revendication 12, la composition solide dans le kit comprenant 245,3 mg de fosaprépitant diméglumine équivalant à 150 mg de fosaprépitant acide libre.

**14.** Kit selon la revendication 12, la solution aqueuse d'albumine sérique humaine dans le kit possédant une concentration d'albumine sérique humaine dans la plage de 0,1 % à 25 % (p/v).

**15.** Kit selon la revendication 12, la composition solide comprenant 245,3 mg de fosaprépitant diméglumine équivalant à 150 mg de fosaprépitant acide libre et une solution aqueuse d'albumine sérique humaine avec la concentration d'albumine sérique humaine dans la plage de 0,1 % à 25 % (p/v).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62370453
- US 62412085
- WO 2014093907 A1
- WO 2016059587 A1


### Non-patent literature cited in the description


- **LEAL AD et al.** *Support Care Cancer,* 2014, vol. 22, 1313-1317
- **LANGFORD P. et al.** *Core Evidence,* 24 September 2009, vol. 5, 77-90
- *CHEMICAL ABSTRACTS,* 170729-80-3
- **COHN EJ et al.** *J. Am. Chem. Soc.,* 1946, vol. 68, 459-475
- **LIN JJ et al.** *Pharmaceutical Research,* 2000, vol. 17, 391-6
- **BOSSE D et al.** *J. Clin. Pharmacol.,* 2005, vol. 45, 57-67
- **CHEN Z et al.** *Biochimica et Biophysica Acta,* 2013, vol. 1830, 5515-5525
- **CHEN RF.** *J. Biol. Chem.,* 1967, vol. 242, 173-181
- **TULLIS.** *JAMA,* 1977, vol. 237, 355-360, 460-463
- **HOUSER et al.** *Surgery, Gynecology and Obstetrics,* 1980, vol. 150, 811-816
- **FINLAYSON.** *Seminars in Thrombosis and Hemostasis,* 1980, vol. 6, 85-120
- **GOODMAN et al.** The Pharmacological Basis of Therapeutics. McGraw-Hill, 1996
- **FEHSKE et al.** *Biochem. Pharmcol.,* 1981, vol. 30, 687-92
- **VORUM.** *Dan. Med. Bull.,* 1999, vol. 46, 379-99
- **KRAGH-HANSEN.** *Dan. Med Bull.,* 1990, vol. 1441, 131-40
- **CURRY et al.** *Nat. Struct. Biol.,* 1998, vol. 5, 827-35
- **SUGIO et al.** *Protein. Eng.,* 1999, vol. 12, 439-46
- **HE et al.** *Nature,* 1992, vol. 358, 209-15
- **CARTER et al.** *Adv. Protein. Chem.,* 1994, vol. 45, 153-203
- *CHEMICAL ABSTRACTS,* 172673-20-0
- *CHEMICAL ABSTRACTS,* 265121-04-8
- Remington's Pharmaceutical Sciences. Lippincon Williams & Wilkins, 2003